(19)

**Europäisches Patentamt**

**European Patent Office**

**Office européen des brevets**

(11) **EP 4 517 763 A1**

(12) **EUROPEAN PATENT APPLICATION**

(43) Date of publication:
05.03.2025 Bulletin 2025/10

(21) Application number: 23306465.8

(22) Date of filing: 04.09.2023

(51) International Patent Classification (IPC):
*G16C 60/00* (2019.01)

(52) Cooperative Patent Classification (CPC):
**G16C 60/00;** G06N 3/045

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Dassault Systèmes**
78140 Vélizy-Villacoublay (FR)

(72) Inventor: **LI, Tianyi**
**Vélizy-Villacoublay (FR)**

(74) Representative: **Bandpay & Greuter**
**11 rue Christophe Colomb**
**75008 Paris (FR)**

(54) **PREDICTING MACROSCOPICAL PHYSICAL PROPERTIES OF A MULTI-SCALE MATERIAL**

(57)     The disclosure relates to a method for training a Deep Material Network-based neural network configured to predict a macroscopical physical property of a multi-scale material. The multi-scale material comprises one or more components. The method comprises obtaining a dataset, each entry of the dataset corresponding to a respective multi-scale material object. The entry comprises a tensor describing the physical property of the object at a macroscopical level, one or more tensors each describing the physical property of a component of the object at a microscopical level, and one or more morphological parameters each describing a morphology of the object. The method further comprises training, based on the dataset, the neural network to predict a tensor describing the physical property of a multi-scale material object at a macroscopical level based on the one or more tensors for the object and based on the one or more morphological parameters for the object.

**Description**

**TECHNICAL FIELD**

The disclosure relates to the field of computer programs and systems, and more specifically to a method, system, and program for prediction a macroscopical physical property of a multi-scale material.

**BACKGROUND**

A number of systems and programs are offered on the market for the design, the engineering, and the manufacturing of objects. CAD is an acronym for Computer-Aided Design, e.g. it relates to software solutions for designing an object. CAE is an acronym for Computer-Aided Engineering, e.g., it relates to software solutions for simulating the physical behavior of a future product. CAM is an acronym for Computer-Aided Manufacturing, e.g., it relates to software solutions for defining manufacturing processes and operations. In such computer-aided design systems, the graphical user interface plays an important role as regards the efficiency of the technique. These techniques may be embedded within Product Lifecycle Management (PLM) systems. PLM refers to a business strategy that helps companies to share product data, apply common processes, and leverage corporate knowledge for the development of products from conception to the end of their life, across the concept of extended enterprise. The PLM solutions provided by Dassault Systèmes (under the trademarks CATIA, ENOVIA and DELMIA) provide an Engineering Hub, which organizes product engineering knowledge, a Manufacturing Hub, which manages manufacturing engineering knowledge, and an Enterprise Hub which enables enterprise integrations and connections into both the Engineering and Manufacturing Hubs. All together the system delivers an open object model linking products, processes, resources to enable dynamic, knowledge-based product creation and decision support that drives optimized product definition, manufacturing preparation, production, and service.

Solutions and products have been proposed for determining mechanical behaviors of materials used in different products and processes. Such solutions and product comprise material constitutive models, and experimental methods. Due to increasing demand for multi-scale materials in various industries, specific solutions and products have been proposed for prediction of mechanical behaviors of such materials.

Document Liu, Wu & Koishi "A deep material network for multiscale topology learning and accelerated nonlinear modeling of heterogeneous materials." Computer Methods in Applied Mechanics and Engineering, 345, 1138-1168, 2019, teaches a data-driven multiscale material modeling method based on mechanistic homogenization theory of representative volume element (RVE) and advanced machine learning techniques. The method uses a collection of connected mechanistic building blocks with analytical homogenization solutions to describe complex overall material responses.

Within this context, there is still a need for an improved prediction of a macroscopical physical property of a multi-scale material.

**SUMMARY**

It is therefore provided a computer-implemented method for training a Deep Material Network (DMN)-based neural network configured to predict a macroscopical physical property of a multi-scale material. The multi-scale material comprises one or more components. The method comprises obtaining a dataset, where each entry of the dataset corresponding to a respective multi-scale material object. The entry comprises a tensor describing the physical property of the multi-scale material object at a macroscopical level. The entry comprises one or more tensors each describing the physical property of a component of the multi-scale material object at a microscopical level. The entry further comprises one or more morphological parameters each describing a morphology of the multi-scale material object. The method further comprises training, based on the obtained dataset, the neural network to predict a tensor describing the physical property of a multi-scale material object at a macroscopical level based on the one or more tensors for the object and based on the one or more morphological parameters for the object.

The method may comprise one or more of the following:

- the Deep Material Network (DMN)-based neural network consists of a first block and a second block; wherein the first block is configured to receive as input the one or more morphological parameters for the object and to output a value of a plurality of network parameters, and the second block is configured to receive as input the value of the plurality of network parameters and the one or more tensors for the object, and to output a prediction of the physical property of the multi-scale material object at a macroscopical level;
- the first block is a feed-forward neural network; and/or the second block has a DMN architecture with the plurality of network parameters;
- the first block is a fully connected neural network;

- the first block consists of a first sub-block and a second sub-block; wherein: the first sub-block is configured to a receive as input a value of a first subset of the one or more morphological parameters and to output a value of a respective subset of the plurality of network parameters, and the second sub-block is configured to a receive as input a value of second subset of the one or more morphological parameters and to output a value of a respective subset of the plurality of network parameters;
- the first subset of the one or more morphological parameters comprises a set of volume fraction for each of the components; and/or the second subset of the one or more morphological parameters comprises one or more orientation parameters;
- the training comprises minimizing a loss function, the loss function penalizing, for each entry, a disparity between: the obtained tensor describing the physical property of the multi-scale material object at a macroscopical level, and the predicted tensor describing the physical property of the multi-scale material object at a macroscopical level; and/or
- the loss function further penalizes a non-respect of a volume fraction constraint for the multi-scale material object and/or a non-respect of a material orientation constraint for the multi-scale material object.

It is further provided a neural network trainable (i.e., learnable) according to the method of training, that is a computer-implemented neural network data structure having the weights of a neural network trained by the method. The provided neural network may for example have been learnt directly by the method, with its weights having been fixed by the training step of the method.

It is further provided a computer-implemented method of use of the neural network trainable according to the method of training. The method of use comprises obtaining one or more tensors each describing the physical property of a component of the multi-scale material object at a microscopical level, and one or more morphological parameters each describing a morphology of the multi-scale material object. The method of use further comprises predicting the macroscopical physical property of the multi-scale material object by applying the neural network on the one or more tensors and the one or more parameters.

It is further provided a computer-implemented method of use of the neural network trainable according to the method of training. The method of use comprises obtaining a tensor describing the physical property of the multi-scale material object at a macroscopical level. The method further comprises determining one or more tensors each describing the physical property of a component of the multi-scale material object at a microscopical level, and one or more morphological parameters each describing a morphology of the multi-scale material object. The determining comprises minimizing a disparity between the obtained tensor and a candidate predicted value for the obtained tensor, the candidate predicted value being determined by applying the neural network on candidate one or more tensors and candidate one or more morphological parameters.

In the method of use, the minimizing of the disparity may comprise computing a gradient of the candidate predicted value with respect to each respective candidate one or more tensors, and a gradient of candidate predicted value with respect to each respective candidate one or more morphological parameters.

It is further provided a database of multi-scale materials. Each entry of the dataset corresponds to a respective multi-scale material. The entry comprises one or more morphological parameters each describing a morphology of the multi-scale material object, and a neural network trained for predicting a macroscopical physical property of a multi-scale material according to the method of training.

It is further provided a computer program comprising instructions for performing the method and/or the method of use.

It is further provided a computer readable storage medium having recorded thereon the computer program and/or the neural network and/or the database.

It is further provided a system comprising a processor coupled to a memory and a graphical user interface, the memory having recorded thereon the computer program and/or the neural network and/or the database.

It is further provided a device comprising the computer readable storage medium having recorded thereon the computer program.

The device may form or serve as a non-transitory computer-readable medium, for example on a SaaS (Software as a service) or other server, or a cloud-based platform, or the like. The device may alternatively comprise a processor coupled to the data storage medium. The device may thus form a computer system in whole or in part (e.g. the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

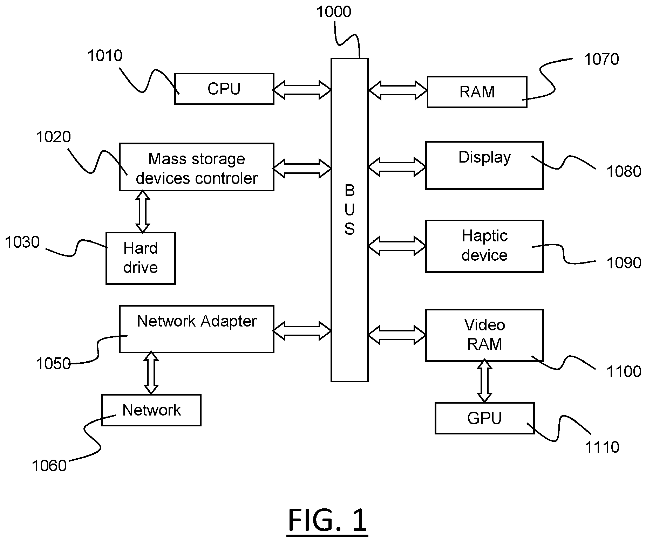

- FIG. 1 shows an example of the system; and
- FIG.s 2-32 show examples of the method.

**DETAILED DESCRIPTION**

It is proposed a computer-implemented method for training (i.e., learning) a Deep Material Network (DMN)-based neural network. The DMN-based neural network is configured to (*i.e.*, trained to) predict a macroscopical physical property of a multi-scale material. The multi-scale material comprises one or more components. The method comprises obtaining a dataset. Each entry of the dataset corresponds to a respective multi-scale material object. The entry comprises a tensor, i.e., a first tensor, describing the physical property of the multi-scale material object at a macroscopical level. The entry further comprises one or more tensors, i.e., second tensor(s). Each of the one or more tensors describes the physical property of a component of the multi-scale material object at a microscopical level. The entry further comprises one or more morphological parameters. Each of the one or more morphological parameters describes a morphology of the multi-scale material object. The method further comprises training, based on the obtained dataset, the neural network to predict a tensor, i.e., a third tensor. The (predicted) tensor describes the macroscopical physical property of a multi-scale material object at a macroscopical level based on the one or more tensor for the object and based on the one or more morphological parameters for the object.

The method may be referred to as "the training method" or "the learning method". The training method trains a neural network to output the third tensor as a prediction (e.g., approximation) of a physical property of a multi-scale material object at a macroscopical level. The training method performs the training by being provided an ensemble of the first tensors. Said first tensors are the ground truth of said physical property of the multi-scale material object at a macroscopical level. The training method performs the training further by being provided an ensemble of the second tensor(s). Said second tensors are the ground truth of said physical property of each component of the multi-scale material object at a microscopical level.

The training method improves the prediction a macroscopical physical property of a multi-scale material given said physical properties of its components. The method obtains this improvement by training a DMN-based neural network architecture. Such an architecture follows the mechanics of the multi-scale material and is able to provide more accurate prediction results. Furthermore, the neural network is further configured to output the prediction based on one or more morphological parameters of the multi-scale material. The morphology of a multi-scale material has a significant role in its macroscopical physical properties of said material. The method is thereby able to train neural networks for a wide range of the materials definable by said one or more morphological parameters.

The method constitutes an improved solution for training neural network of the DMN-type compared to the cited prior art. As the prior art discloses the training for a given (i.e., fixed) morphology (which is defined by the one or more morphological parameters). Since the trained parameters of the neural network depend on said given morphology, it is required to re-train the neural network for each new morphology which is computationally costly, or interpolate/extrapolate outputs of trained network for a new morphology which deteriorates the accuracy of the prediction. The method of current disclosure, however, integrates the morphological parameters into the training stage (which is offline), thereby significantly reduces the computational cost for each new morphology at the deployment/inference stage (which is online). The method provides these benefits without sacrificing the accuracy of the prediction.

The method thereby serves to train neural networks which are able to predict a macroscopical physical property of a multi-scale material consisting of various components and in different morphologies. Such a prediction is an advantageous solution compared to traditional mechanical tests to experimentally determine said mechanical properties. Such trained neural networks provide a designer with the ability to obtain a prediction of the mechanical property in a non-destructive way, with high precision and in a significantly shorter time compared to said mechanical tests. This enables the designer to assess much wider class of materials for a particular use case, thereby improving the final design. The use case may be defined for a mechanical object formed, at least partially, in the multi-scale material. The use case may further be defined by a set of load cases supposed to be applied on said mechanical object. The set of load cases may be a representation of forces, torques, and/or stresses on the mechanical object upon its functioning.

The method and/or the trained neural network may be used for designing a multi-scale material object or product and/or manufacturing the object or product in the real-world further to its design. The object may be an additive manufacturable part, i.e. a part to be manufactured by additive manufacturing (i.e., 3D printing). Additive manufacturing methods are in particular well adapted to manufacture objects in a multi-scale material as these methods are capable to precisely fabricate the internal microstructure of said objects.

For example, the trained neural network may be used to predict a macroscopical physical property of a given multi-scale material object (such as a mechanical property of the object at a macroscopic level, e.g., a compliance or stiffness) based on one or more tensor(s) each describing the physical property of a component of the multi-scale material object at a microscopical level (such as a mechanical property of each component at a microscopic level, e.g., a compliance or stiffness) and on one or more morphological parameters (e.g., a volume fraction, a misalignment of fibers, and/or an aspect ratio of fibers) each describing a morphology of the multi-scale material object, for example according to the first aspect of the method of use discussed hereinbelow. This may be done (and for example repeated for various inputs and/or for predicting different properties) during the design of the object, for example to test various inputs to converge toward a desired microphysical property for the object.

Alternatively, the trained neural network may be used to optimize the inputs (*i.e.* the one or more tensors each describing the physical property of a component of the multi-scale material object at a microscopical level (such as a mechanical property of each component at a macroscopic level, e.g., a compliance or stiffness), and one or more morphological parameters (e.g., a volume fraction, a misalignment of fibers, and/or an aspect ratio of fibers) each describing a morphology of the multi-scale material object) to reach a desired (provided) tensor describing the physical property of the multi-scale material object at a macroscopical level (such as a mechanical property of the object at a macroscopic level, e.g., a compliance or stiffness), for example according to the second aspect of the method of use discussed hereinbelow. This allows finding the optimal inputs of the network to reach a provided optimal tensor describing the physical property of the multi-scale material object at a macroscopical level.

The method and/or the use of the neural network may be included in a design and manufacturing process. Such process can include:

- optionally, performing the training method and/or providing the neural network obtained according to the training method;
- designing a multi-scale material object, for example by using the trained neural network according to (*e.g.*, repetitions or iterations) of the first aspect or the second aspect of the method of use, to test various inputs of the network (i.e., the tensors describing the physical property at the macroscopical and/or the morphological parameters) and/or to determine optimal inputs for a desired output (i.e., the optimal parameters and/or the optimal tensors describing the physical property of the components at the microscopical for a desired tensor describing the physical property at the microscopical level); and
- using the outcome of the design to manufacture the object in the real-world.

According to the type of the system used for the design, the outcome of the design may be defined by different kinds of data. The system may indeed be any combination of a CAD system, a CAE system, a CAM system, a PDM system and/or a PLM system as known in the field. Designing a CAD model is a first step towards a computer-aided manufacturing. Indeed, CAD solutions provide key functionalities, such as feature based modeling and boundary representation (B-Rep), to reduce the risk of errors and the loss of precision during the manufacturing process handled with a CAM solution. Indeed, a CAD model is intended to be manufactured. Therefore, it is a virtual twin, also called digital twin, of an object to be manufactured with two objectives:

- checking the correct behavior of the object to be manufactured in a specific environment (e.g., in combination with the first or the second aspect of the method of use to ensure a desired physical property for a design); and
- ensuring the manufacturability of the object to be manufactured.

The using of the outcome of the design may be included in a production process, which may comprise, after obtaining the design, producing a physical product corresponding to the outcome of the design inputted to the production process. The production process may comprise the following steps:

- (e.g. automatically) obtaining a CAD model or a CAE model of the design (e.g., upon one or more transformation/-conversion);
- optionally, (e.g. automatically) converting the obtained CAE model into a CAD model, using a (e.g. automatic) CAE to CAD conversion process;
- using the obtained CAD model for manufacturing the design.

Using the CAD model for manufacturing may for example comprise the following steps:

- editing the obtained CAD model;
- performing simulation(s) based on the CAD model or on a corresponding CAE model (*e.g.* the CAE model from which the CAD model stems, after a CAE to CAD conversion process), such as simulations for validation of mechanical, use and/or manufacturing properties and/or constraints (*e.g.* structural simulations, thermodynamics simulation, aerodynamic simulations);
- editing the CAD model based on the results of the simulation(s);
- optionally (*i.e.* depending on the manufacturing process used, the production of the mechanical product may or may not comprise this step), (*e.g.* automatically) determining a manufacturing file/CAM file based on the (*e.g.* edited) CAD model, for production/manufacturing of the manufacturing product;
- sending the CAD file and/or the manufacturing file/CAM file to a factory; and/or

- (*e.g.* automatically) producing/manufacturing, based on the determined manufacturing file/CAM file or on the CAD model, the mechanical product originally represented by the model outputted by the design method. This may include feeding (*e.g.* automatically) the manufacturing file/CAM file and/or the CAD file to the machine(s) performing the manufacturing process.

This last step of production/manufacturing may be referred to as the manufacturing step or production step. This step manufactures/fabricates the part/product based on the CAD model and/or the CAM file, *e.g.* upon the CAD model and/or CAD file being fed to one or more manufacturing machine(s) or computer system(s) controlling the machine(s). The manufacturing step may comprise performing any known manufacturing process or series of manufacturing processes, for example one or more additive manufacturing steps, one or more cutting steps (e.g. laser cutting or plasma cutting steps), one or more stamping steps, one or more forging steps, one or more bending steps, one or more deep drawing steps, one or more molding steps, one or more machining steps (e.g. milling steps) and/or one or more punching steps. Because the design method improves the design of a model (CAE or CAD) representing the part/product, the manufacturing and its productivity are also improved.

The training method is now discussed.

As known from the field of machine-learning, a neural network is a function comprising operations according to an architecture, each operation being defined by data including parameters (also known as weight values). The architecture of the neural network defines the operand of each operation and the relation between said parameters. The learning of a neural network thus includes determining values of the weights based on a dataset configured for such learning and corresponding to its architecture. In other words, training a neural network obtains a determinative function relating input(s) of said network to corresponding output(s). Such a training is performed using a training dataset comprising of a set of input(s) with a corresponding ground truth output(s). The training dataset includes data pieces each forming a respective training sample. The training samples represent the diversity of the situations where the neural network is to be used after being learnt. Any dataset referred herein may comprise a number of training samples higher than 1000, 10000, 100000, or 1000000.

By a "Deep Material Network (DMN)- based neural network", it is meant a neural network which its architecture includes a sub-architecture (i.e., a part of said architecture) according to DMN. Such a DMN sub-architecture may be any DMN architecture known in the field, for example the DMN sub-architecture may be the deep material network architecture of the document Liu et al., (2019). A deep material network for multiscale topology learning and accelerated nonlinear modeling of heterogeneous materials. Computer Methods in Applied Mechanics and Engineering, 345, 1138-1168, which is incorporated herein by reference.

As known in the field of mechanics and material science, by a "multi-scale material" (which may be also equivalently called composites, architectured materials, or metamaterials) it is meant a heterogeneous material containing a microstructure. A heterogeneous material is composed of multiple materials which are spatially arranged in an internal structure, i.e., microstructure. The multi-scale material comprises one or more components. In other words, the microstructure of the multi-scale material object defines the topology and the geometry of respective parts of the object formed in each component. Each of the one or more components may be a heterogeneous or homogeneous material. The multi-scale material may be a parametric multi-scale material, i.e., a material with a morphology/microstructure defined by (a set of) the one or more (morphological) parameters. Each set of values for the one or more parameters defines (a realization of) a multi-scale material. In other words, each multi-scale material object is a realization of the parametric multi-scale material with a value for the set of parameters. By a "multi-scale material object" it is meant a physical object which is formed in the multi-scale material. Such a multi-scale material object may have a standard geometry and dimensions, for example according to a testing standard.

The method obtains a dataset. Each entry of the dataset corresponds to a respective multi-scale material object. By obtaining a dataset it is meant providing a dataset. The method may obtain the dataset by accessing a local storage on which the dataset is stored (e.g., in a database format), or by a remote access to a data storage service, for example on a cloud storage. The method may create said dataset (for example in a dataset forming step) before providing the dataset to the method.

Each entry comprises a tensor which describes the physical property of the multi-scale material object at a macroscopical level. Each entry further comprises one or more tensors each describing the physical property of a component of the multi-scale material object at a microscopical level. By "physical property of a multi-scale material object" it is meant any constituent property that defines the physical behavior of an object formed in said multi-scale material. The physical property may be a mechanical, a thermal, a magnetic, or an electric property. A mechanical property may be a compliance or a stiffness. A thermal/electrical property may be a thermal/electrical conductivity, while a magnetic property may be a magnetic permeability. By a physical property of a component of a multi-scale material object at a microscopic level it is meant a respective physical property that can describe the physical behavior of said component at the microscopic level. By a physical property of a multi-scale material object at a macroscopic level it is meant a respective equivalent or effective physical property that can describe the physical behavior of said object at the macroscopic level, i.e., the object as whole. As known in mathematics (see https://en.wikipedia.org/wiki/Tensor), a tensor is an algebraic object that describes a multilinear relationship between sets of algebraic objects related to a vector space. Tensors may map between different objects such as vectors, scalars, or other tensors. A tensor may be represented as an array (e.g., a multidimensional array) consists of one or more components. Tensors may be of various types. For examples, scalars or vectors are examples of tensors. Thereby, by a tensor describing the physical property (at macroscopic or microscopic level) it is meant a tensor the components of which representing said physical properties. For example, when the physical property is compliance or stiffness, each component of the tensor describing the compliance or stiffness may include one or more linear elastic properties. The linear plastic properties may be Young's modulus (that describes the stiffness required per unit extension in a certain direction), shear modulus (that describes the shear stiffness required per unit shear deformation), and/or Poisson ratio (that describes the relative deformation between two orthogonal directions).

Each of the macroscopical level tensor and the one or more microscopic level tensors may be obtained according to an experiment (e.g., in respective standard conditions) or a high-fidelity numerical method, for example a finite element method. Such tensors may be computed before performing the method and stored in the database to be provided to the method.

The entry may further comprise one or more morphological parameters. Each of the one or more morphological parameters describes a morphology of the multi-scale material object. By the morphology of the multi-scale material object it is meant a qualitative description of the microstructure of said multi-scale material.

The method then trains the neural network based on the obtained dataset. By "based on the obtained dataset", it is meant that the dataset is a training dataset of the neural network. The trained neural network is configured to predict a tensor describing the physical property of a multi-scale material object at a macroscopical level based on the one or more tensors describing the physical property at a microscopical level, and based on the one or more morphological parameters for the object. In other words, the (trained) neural network receives, as input, the one or more tensors describing the physical property at a microscopical level, and the one or more morphological parameters for the object, and predicts (i.e., outputs) said tensor. By being configured to predict said tensor it is meant that the neural network outputs a tensor which approximates a (ground truth) physical property of the multi-scale material object at a macroscopical level. The method may train the neural network according to any known method in the field of machine learning and artificial intelligence.

Examples of the training method are now discussed.

In examples, the Deep Material Network (DMN)-based neural network may consist of a first block and a second block. In other words, the first and the second block are nor overlapping and forms a partitioning of the DMN-based neural network. The first block is configured to receive as input the one or more morphological parameters for the object and to output a value of a plurality of network parameters (also called DMN-fitting parameters). The second block is configured to receive as input the value of the plurality of network parameters (i.e., the output of the first block) as well the one or more tensors for the object. The second block is further configured to output a prediction of the physical property of the multi-scale material object at a macroscopical level. By the "plurality of the network parameters" it is meant the plurality of parameters which define the relationship between output of the second block and the inputted one or more tensors to the second block. Thereby, the architecture of the network provides a dependence of the parameters of the second block on the output of the first block which is itself dependent on the one or more morphological parameters. This constitutes an improved training compared to standard DMNs in which the parameters of the network are to be trained for given (i.e., fixed) morphological parameters and thereby are not applicable to different morphologies of a multi-scale material object. Furthermore, these examples provide a separation between the first block and the second block. In other words, in these examples, the first block is configured to output the network parameters without being inputted the one or more tensors for the object, and only using the morphological parameter(s). On the other hand, the second block is configured to output the physical property at a macroscopical level without being inputted the one or more morphological parameters. Such a separation enables the neural network to use more efficient architectures for the unknowns in each block (i.e., the network parameters in the first block, and the predicted tensor for the physical property in the second block). For example, the second block may comprise a DMN architecture which has better accuracy for such physical property predictions.

In examples, the first block may be a feed-forward neural network. As known per se by a "feed-forward neural network" it is meant a neural network in which the flow is unidirectional from the input nodes, through the hidden nodes (if any) and to the output nodes, without any cycles or loops (see https://en.wikipedia.org/wiki/Feedforward neural network). Alternatively or additionally, the second block may be (i.e., consists of) a DMN architecture with the plurality of network parameters. In such cases, the plurality of network parameters may comprise a plurality of weights and a plurality of rotations for the DMN architecture. Each rotation of the neural network may be a rotation tensor (e.g., a 3D rotation tensor). Such rotations and weights may be for example be according to the already cited Document Liu et al., (2019). A deep material network for multiscale topology learning and accelerated nonlinear modeling of heterogeneous materials. Computer Methods in Applied Mechanics and Engineering, 345, 1138-1168.

In examples, the first block may be a fully connected neural network. In other words, in such examples, each of the plurality of network parameters are defined on the whole of the one or more morphological parameters.

Alternatively, and in other examples, the first block may consist of a first sub-block and a second sub-block. The first sub-block is configured to receive as input a value of a first subset of the one or more morphological parameters and to output a value of a respective subset of the plurality of network parameters. The second sub-block is configured to a receive as input a value of second subset of the one or more morphological parameters and to output a value of a respective subset of the plurality of network parameters. In other words, in such examples, each subset of the plurality of network parameters is defined on a respective subset of the one or more morphological parameters. The first subset of the one or more morphological parameters and the second subset of the one or more morphological parameters form a decomposition or partitioning of (the set of) one or more morphological parameters. Similarly, the subset of the plurality of network parameters respective to the first subset and the subset of the plurality of network parameters respective to the second subset form a decomposition or partitioning of the plurality of network parameters. In other words, the union of a first subset and a second subset, for each of the morphological parameters or the network parameters equals the whole set of parameters, while an intersection of said first subset and said second subset is void.

In examples, the first subset of the one or more morphological parameters comprises a set of volume fraction for each of the components. Alternatively or additionally, the second subset of the one or more morphological parameters comprises one or more orientation parameters. By orientation parameters it is meant the parameters which define one or more orientations of the microstructure of the multi-scale material, for example orientation tensors, for example relative orientations between the components. In examples where the second block has a DMN architecture with the plurality of network parameters, the subset of the plurality of network parameters respective to the first subset comprises weights of the DMN. Further alternatively or additionally, the subset of the plurality of network parameters respective to the second subset comprises rotations of the DMN.

In examples, the training comprises minimizing a loss function. The loss function penalizes, for each entry, a disparity between the obtained tensor describing the physical property of the multi-scale material object at a macroscopical level, and the predicted tensor describing the physical property of the multi-scale material object at a macroscopical level. The disparity may be measured in a mathematical norm. The loss function may be of the type:

$$\mathcal{L} = \frac{1}{|\mathbb{P}|}\sum_{\mathbb{P}} \mathcal{L}_p, \qquad \mathcal{L}_p = \frac{1}{|\mathbb{M}|}\sum_{\mathbb{M}} e_i^2, \qquad e_i = \frac{\|\overline{\mathbb{C}}_i^{DMN} - \overline{\mathbb{C}}_i^{FE}\|}{\|\overline{\mathbb{C}}_i^{FE}\|}.$$

Here the loss $e_i$ is based on the Mean Squared Error between the predicted tensor $\overline{\mathbb{C}}_i^{DMN}$ and a ground truth tensor $\overline{\mathbb{C}}_i^{FE}$ generated (i.e., obtained) by FE-RVE simulations. The notation $|\mathbb{P}|$ denotes the number of samples in the parametric space, i.e., the one or more morphological parameters. The notation $|\mathbb{M}|$ denotes the number of samples in the material space, i.e., the one or more obtained tensors. The errors (i.e., disparities) among material samples $\mathbb{M}$ are then averaged to compute the loss $\mathcal{L}_p$ at a fixed microstructural parameter value. Finally, they are averaged, among samples $\mathbb{P}$ in the parametric space, to yield the total loss function $\mathcal{L}$,. Any of the samples in the material space and the samples in the parametric space may be obtained according to any sampling method, for example, a Latin hypercube method. By being "of the type" of a mathematical formula for an identity it is meant that said identity is exactly given by the said formula, or by said formula up to a scaling and/or regularization terms.

The loss function may further penalize a non-respect of a volume fraction constraint for the multi-scale material object. This penalization means that the training of the neural network tends to match the volume fraction in the DMN architecture with the real (i.e., ground truth) volume fraction value of the microstructure for arbitrary volume fraction values. Alternatively or additionally, the loss function may further penalize a non-respect of a material orientation constraint for the multi-scale material object. This penalization means that the training of the neural network tends to match the average material frame orientation predicted by DMN to the real microstructure.

In examples where the loss function penalizes both of the non-respect of the volume fraction and the non-respect of a material orientation constraint, the loss function may be of the type:

$$\mathcal{L} = \frac{1}{|\mathbb{P}|}\sum_{\mathbb{P}} \mathcal{L}_p + \mathcal{L}_{vf} + \mathcal{L}_a,$$

in which $\mathcal{L}_p$ is the loss function as defined above, $\mathcal{L}_{vf}$ is the respective term of the loss function which penalizes the non-respect of the volume fraction (denoted as *vf*), and $\mathcal{L}_a$ is the respective term of the loss function which penalizes the non-respect of a material orientation.

It is further provided, a neural network trainable according to the method of training, *i.e.*, a neural network having weight values that are identical to those that would result from the training according to the training method. Such a neural network may be any DMN-based neural network which is configured to predict a tensor describing the physical property of a multi-scale material object at a macroscopical level based on the one or more tensors for the physical property of a component of the multi-scale material object at a microscopical level, and based on the one or more morphological parameters for the object. Such a neural network may be having been trained according to any examples of the method of training, i.e. a neural network having weight values that directly result from the training according to the training method.

It is further provided a computer-implemented method of use of the neural network. Such a method of use comprises obtaining one or more tensors each describing the physical property of a component of the multi-scale material object at a microscopical level, and one or more morphological parameters each describing a morphology of the multi-scale material object. The method further comprises predicting the macroscopical physical property of the multi-scale material object by applying the neural network (e.g., a neural network trained according to the training method) on the one or more tensors and the one or more parameters. In this first aspect, the method of use forms a direct prediction method for the physical property of the multi-scale material at the macroscopic level. Alternatively, or additionally, the method of use comprises obtaining a tensor describing the physical property of the multi-scale material object at a macroscopical level, and determining one or more tensors each describing the physical property of a component of the multi-scale material object at a microscopical level, and one or more morphological parameters each describing a morphology of the multi-scale material object. In this second aspect the method of use forms an inverse identification method. In such an inverse identification method, the method determines the physical properties of the one or more components of a multi-scale material and the one or more morphological parameters based on a given physical property of the multi-scale material object at a macroscopical level (via the obtained tensor). The determining comprises minimizing a disparity between the obtained tensor and a candidate predicted value for the obtained tensor. The candidate predicted value is determined by applying the neural network (e.g., a neural network trained according to the training method) on candidate one or more tensors and candidate one or more morphological parameters. In other words, the method performs the minimizing by (e.g., iteratively) searching over (a set of) candidates for the one or more tensors and (a set of) candidates for the one or more morphological parameters, apply the trained neural network on each group of candidates for the one or more tensors and the one or more morphological parameters to obtain the candidate predicted value for the obtained tensor and thereby the disparity to be minimized. In yet other words, the trained neural network is part of the objective function (i.e., the disparity) of such a minimization and the one or more tensors and the one or more morphological parameters are the free variables of such a minimization. The disparity may be equivalently called as loss function. In practice, this aspect may form or be a part of an identification process in which the macroscopic physical property of a multi-scale material object is known (e.g., via a high-fidelity numerical simulation like finite element method or via an experiment).

The method of use may comprise both of the aspects discussed above or any of them independently. In other words, when a neural network is trained according to the training method, it can be used for direct prediction of macroscopic physical property of a multi-scale material object, and/or for an inverse identification of the physical properties of the component(s).

In any of the two aspects of the method of use discussed above, the input and output of the network during the online phase of use (i.e., inference or deployment) are of the same type as of the respective part of an entry in the obtained dataset used in the offline stage of training according to the method of training. In other words, the obtained tensor related to a macroscopical level, the obtained one or more tensors related to a microscopical level, and the obtained one or more morphological parameters are of the same type (i.e., in physical nature, and/or in units) as the tensor describing the physical property of the multi-scale material object at a macroscopical level, the one or more tensors related to a microscopical level, and the one or more morphological parameters of each entry of the provided dataset in the method of training, respectively.

The disparity may be a norm of difference between the obtained tensor and the predicated value thereof. In examples, the disparity may be of the type:

$$\mathcal{L}_{cal} = \frac{\parallel \overline{\mathbb{C}}_{DMN} - \overline{\mathbb{C}}_{Data} \parallel^2}{\parallel \overline{\mathbb{C}}_{Data} \parallel^2}$$

where $\mathcal{L}_{cal}$ is the disparity, $\overline{\mathbb{C}}_{Data}$ is the obtained tensor, and $\overline{\mathbb{C}}_{DMN}$ is the predicted value of the obtained tensor. Here, the norm $\parallel . \parallel$ represents standard Frobenius norm.

The minimizing of the disparity may comprise computing a gradient of the candidate predicted value with respect to each respective candidate one or more tensors, and a gradient of candidate predicted value with respect to each respective candidate one or more morphological parameters. In other words, the minimizing is a gradient-based minimization. The method may compute each of the gradient of the candidate predicted value with respect to each respective candidate one or more tensors and a gradient of candidate predicted value with respect to each respective candidate one or more morphological parameters using automatic differentiation according to the architecture of the DMN-based framework.

It is further provided a database of a multi-scale materials. Each entry in such a database corresponding to a respective multi-scale material. Each entry comprises one or more morphological parameters each describing a morphology of the multi-scale material object. Each entry further comprises a neural network trained for predicting a macroscopical physical property of a multi-scale material according to the method of training. Each entry may be in particular related to a multi-scale material object formed in said respective multi-scale material. Such a multi-scale material object may have a standard geometry and dimensions, for example according to a testing standard.

This constitutes an improved solution to store a database of multi-scale materials. As discussed above, multi-scale materials have a microstructure which needs to be represented in the storage. Known solutions in the field stores the multi-scale material using unstructured meshes that discretize spatially the microstructure. Storage of this microstructure for practical ranges of microscopic-level properties of the components and for different morphologies thereby is significantly memory costly. The database provided is thereby an improved solution as it only stores, in respect to each multi-scale material, a trained network.

By "database", it is meant any collection of data (i.e. information) organized for search and retrieval (*e.g.*, a relational database, e.g. based on a predetermined structured language, *e.g.*, SQL). When stored on a memory, the database allows a rapid search and retrieval by a computer. Databases are indeed structured to facilitate storage, retrieval, modification, and deletion of data in conjunction with various data-processing operations. The database may consist of a file or set of files that can be broken down into records, each of which consists of one or more fields. Fields are the basic units of data storage. Users may retrieve data primarily through queries. Using keywords and sorting commands, users can rapidly search, rearrange, group, and select the field in many records to retrieve or create reports on particular aggregates of data according to the rules of the database management system being used.

As discussed above, in examples that the DMN-based neural network consists of a first block and a second block, the first block is configured to output the network parameters without being inputted the one or more tensors for the object, and only by using the morphological parameter(s). In other words, the parameters of the trained second block (i.e., the network parameters) are obtainable from the plurality of morphological parameters and the first block. In such examples, the database of the multi-scale material may, instead of the whole trained neural network (i.e., the first block and the second block) only stores the first block. This significantly improves the required storage.

The methods are computer-implemented. This means that steps (or substantially all the steps) of the methods are executed by at least one computer, or any system alike. Thus, steps of the methods are performed by the computer, possibly fully automatically, or, semi-automatically. In examples, the triggering of at least some of the steps of the methods may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g., one for the program, and possibly one for the database).

FIG.1 shows an example of the system, wherein the system is a client computer system, *e.g.* a workstation of a user.

The client computer of the example comprises a central processing unit (CPU) 1010 connected to an internal communication BUS 1000, a random-access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

An implementation of the methods discussed above are now discussed.

The implementation is related to a physics-informed AI surrogate which is capable of predicting effective behaviors of materials with a particular fixed microstructure. More specifically, the implementation may be related to a prediction of the effective physical properties of these materials when the microstructure itself can vary.

The implementation is related to the numerical simulation of the effective macroscopic mechanical behavior of heterogeneous materials containing a microstructure. The heterogeneity implies that multiple materials are spatially arranged in an internal structure, i.e., a microstructure. Such materials are often called composites, architectured materials, metamaterials, and multiscale materials. Such achitectured materials or metamaterials also include lattice or spinodal-like structures, in which only one material is organized with a structure. The other material phase is the void. These multiscale materials are frequently used in various industrial applications, such as automobile, aerospace, medical, civil engineering, etc.

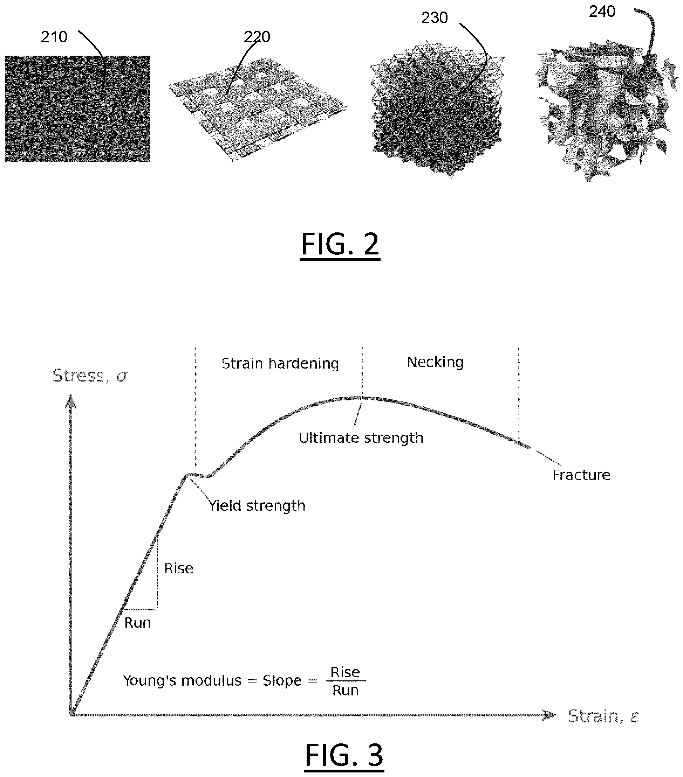

FIG. 2 represents examples of architectured materials. Example 210 is a unidirectional fiber composite, example 220 is a woven composite, example 230 is a lattice structure, and example 240 is a spinodal structure.

The multiscale nature of these materials originates from the fact that the macroscopic behavior is determined both by the constituent properties (mechanical, thermal, or other physical properties) and by the morphology (geometry, topology, etc.) of the microstructure. For instance, the mechanical behavior is often described by a stress-strain curve, which measures the mechanical stress required for certain amount of deformation of the material.

FIG. 3 presents an example of the strain-stress curve. The initial slope, called Young's modulus, characterizes the elastic stiffness. In the case of a multiscale material, the stress-strain curve depends on the material properties of its constituents as well as the microstructure morphology.

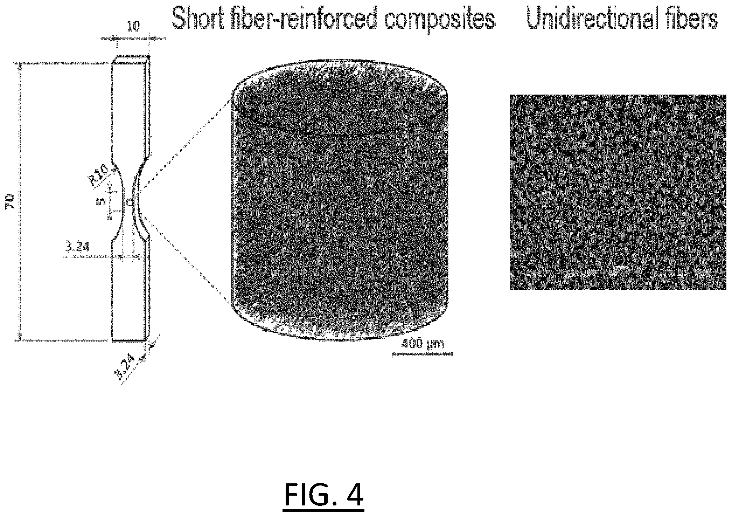

One of such heterogeneous materials is the fiber-reinforced composites, where glass or carbon fibers are used as reinforcement in a polymer matrix. They can guarantee high mechanical resistance specifications while achieving overall part weight reductions. FIG. 4 shows an examples of such materials.

The microstructure of such multiscale material can be described by several microstructural parameters that characterize its morphology.

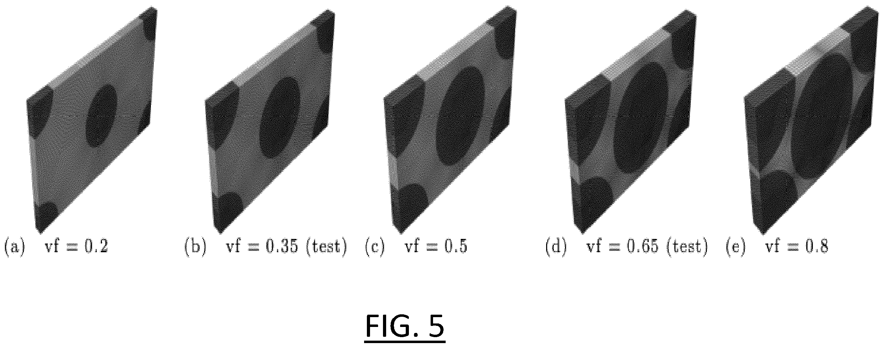

FIG. 5, for a unidirectional fiber composite, represents volume fractions $vf$ (of the fibers) which varies from 0.2 to 0.8.

Another morphological parameter for such unidirectional fiber composites is the fiber misalignment. Due to manufacturing process, fibers are not perfectly aligned but present some misalignment. This misalignment can be described by the initial misalignment angle.

FIG. 6 presents an example of such misalignment among the fibers. FIG. 6 presents three fiber composites with three different misalignment angles: (top) 0°, (middle) 1° and (down) 2°. The implementation may take this morphological parameter into account if its influence on the effective physical properties is to be studied.

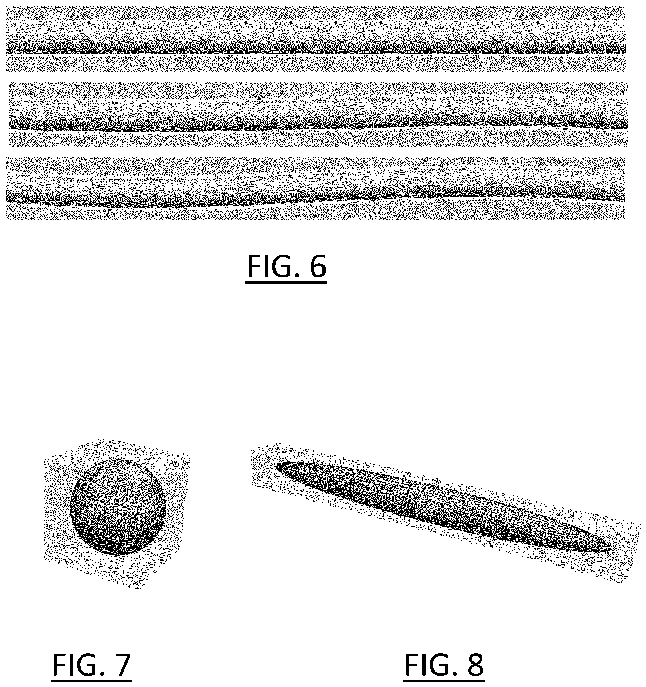

Short-fiber reinforced plastic composites can be described by the volume fraction of the fibers, aspect ratio of the fibers, fiber orientation, etc. The aspect ratio (ar) is a scalar defined as the ratio between the length and the diameter. This parameter characterizes the elongation of the fibers and in general varies from 1 (near spherical particles) to over 100 (very elongated fibers). FIG.s 7 and 8 present examples of two values of the aspect ratio. In FIG. 7, ar=1 and fibers are like spheres, while in FIG. 8, ar=10 and the fibers are more elongated.

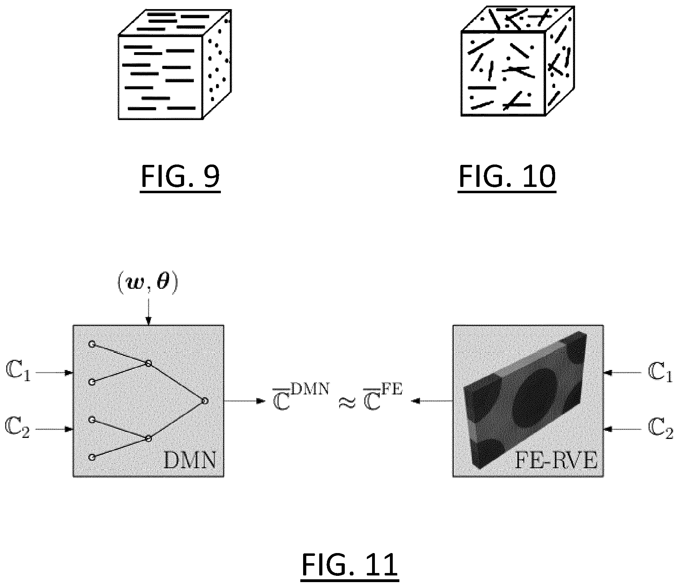

Fiber orientation can be characterized by a vector $(a_x, a_y, a_z)$ with the property $a_x + a_y + a_z = 1$. Each of these three components $a_i$ describes the statistical probability of finding fibers in the $i$ direction. FIG.s 9 and 10 present examples. In FIG. 9, all fibers are oriented in the $X$ direction and fiber orientation is described by the vector (1, 0, 0). In FIG. 10, the fibers are randomly oriented in the 3D space, and fiber orientation is described by the vector $\left(\frac{1}{3}, \frac{1}{3}, \frac{1}{3}\right)$.

Such multiscale materials with associated microstructural parameters are called parametric multiscale materials. The implementation is related to predict the effective mechanical properties of such parametric multiscale materials. Accurate and fast predictions of effective physical behaviors is required to design optimal metamaterials for industrial applications, to quantify the uncertainties in the material properties, and to inversely identify the properties of its constituents. Efficient scale bridging techniques between macroscopic and microscale simulations is also needed, to design and validate industrial components made of a multiscale material.

The implementation proposes a new neural network architecture for generic parametric microstructures, with varying volume fractions and possibly other geometrical parameters. The implementation using a DMN formulation for a fixed microstructure while being able to predict accurately and efficiently the effective linear and nonlinear behaviors, at arbitrarily given microstructural parameters.

The implementation is based on the Deep Material Network (DMN) formulation. Such a formulation can be regarded as a neural network-like architecture approximating the effective elasticity tensor $\overline{\mathbb{C}}$ of a heterogeneous material, given the elasticity tensors of its constituents $(\mathbb{C}_1, \mathbb{C}_2)$. The fitting parameters of DMN are the weights and the rotations of the internal neurons, and are denoted by ($w, \theta$). During the offline training procedure, these parameters are adjusted so that the predicted $\overline{\mathbb{C}}$ of DMN match the values computed by computational homogenization techniques, such as the finite-element method applied on a representative volume element of the microstructure (FE-RVE). FIG. 11 presents a schematic representation of a DMN in comparison to a finite element solution.

The original DMN formulation is limited to a particular fixed microstructure. The trained fitting parameters are learnt to be a compact representation of a microstructure in particular. If the morphology of the microstructure varies with certain microstructural parameters $p$, it can be expected ($w, \theta$) should also vary in a certain way with $p$. However, with the original DMN formulation, the fitting parameters ($w, \theta$) are fixed.

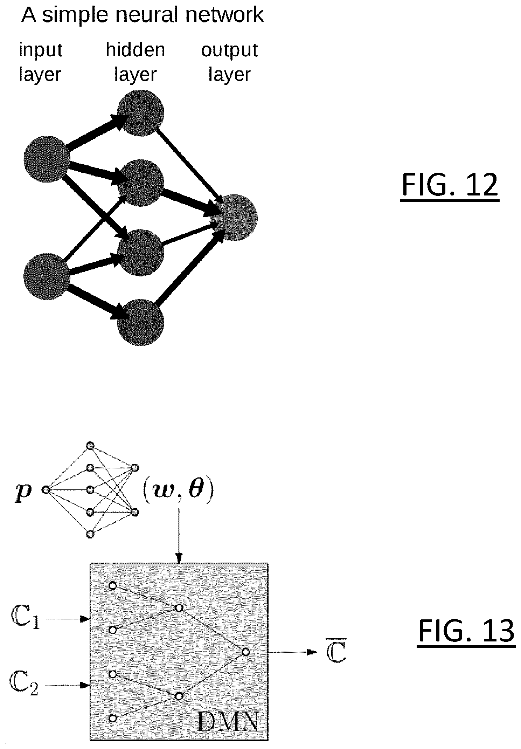

To resolve this issue, this implementation proposes to use a standard feedforward neural network to account for the dependence of DMN fitting parameters ($w, \theta$) on the microstructural parameters $p$. This neural network is composed of an input layer, which takes the microstructural parameters $p$, and of an output layer which computes the DMN fitting parameters ($w, \theta$). The hidden layers are composed of affine functions and nonlinear activation functions. FIG. 12 presents a simple example of such feedforward neural networks.

The neural network architecture of the implementation is hence composed of two parts. An example of such a two-part neural network is presented in FIG. 13. In this example, the implementation provides a parameter vector $p$ representing the microstructure features of the multiscale material (volume fraction, fiber length, fiber diameter, fiber orientation, etc.) to a first neural network. The first neural network is configured to predict a compact representation ($w, \theta$) of a particular microstructure. Using the computed compact representation, the implementation then computes the effective elasticity tensor of the microstructure using the original formulation of DMN.

The forward function that predicts the effective properties $\overline{\mathbb{C}}$ can be described by the following procedure:

- Provide a parameter vector $p$ representing the microstructure features of the multiscale material, e.g., volume fraction, or fiber orientation.
- Using this parameter vector $p$ as input, the first neural network predicts the compact representation ($w, \theta$) of this particular microstructure with given parameters.
- Provide the linear elastic properties (Young's moduli, shear moduli and Poisson ratios) of each constituent, represented by the elasticity tensors $(\mathbb{C}_1, \mathbb{C}_2)$.
- The original DMN model takes ($w, \theta$) as input to predict macroscopic properties of the multiscale material $\overline{\mathbb{C}}$.

The implementation adopts a generic feedforward neural network to account for the dependence of DMN parameters (i.e., compact representation of microstructures) on microstructural ones. It is hence generalizable for arbitrary parametric microstructures with multiple parameters of different natures. In particular, the implementation exploits Physics-Informed Neural Networks (PINNs). In such network, compared to standard feedforward neural networks, the architecture itself is designed such that it satisfies certain properties justified by physics. Furthermore, additional micromechanics-based constraints are included to improve generalization capability of the neural network architecture. The obtained neural network is thus capable of predicting effective properties with high accuracy and efficiency in the parametric space.

The offline training process of the implementation may be done simultaneously, by using the dataset generated from multiple instances (different parametric values) of a parametric microstructure. This guarantees that the proposed neural network can be trained efficiently with all the information at hand. The offline training procedure can be described as follows:

- Provide a Design of Experiments (sampling points) in the parametric space describing different microstructural parameters of the microstructure. In the example of a microstructure that only contains one volume fraction parameters, the parametric space is hence a one-dimensional interval $[vf_{min}, vf_{max}]$.

- Provide a Design of Experiments (sampling points) in the material space that describe different input material properties $(\mathbb{C}_1, \mathbb{C}_2)$.

- Perform finite-element simulations for each combined sampling points in the parametric space and in the material space.

- Define a loss function that compares the prediction of parametric DMN model and the previous dataset.

- Adjust the fitting parameters of parametric DMN model in order that DMN can approximate well the previous dataset. This is done in an iterative fashion. For each epoch (i.e., iteration), the fitting parameters are incrementally adjusted according to the gradient of the loss function with respect to each fitting parameter.

The architecture according to the implementation also provides a new compact representation of parametric microstructures with an arbitrary number of parameters. The reduction in the storage requirement is significant, as a finite number of parameters are now able to represent an infinite number of complex microstructures three-dimensional meshes.

The implementation may also be used in an inverse identification problem setting. Given the effective properties of the heterogeneous material which can be measured experimentally, its input constituent properties can now be efficiently calibrated or identified using the implementation. The procedure of such an inverse identification problem can be described as follows:

- Train a parametric DMN representing the given parametric microstructure.
- Provide the effective properties of the composite.
- Inversely identify the input constituent and microstructural parameters.

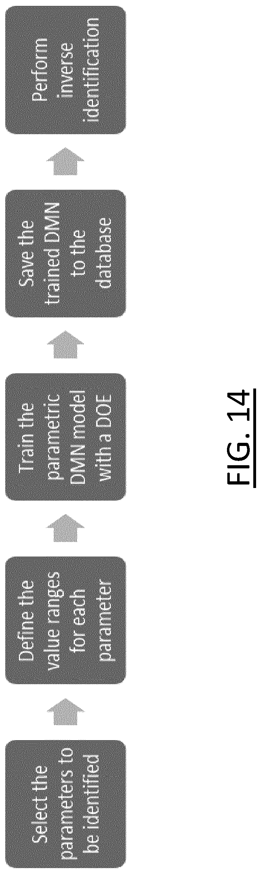

FIG. 14 presents an example flowchart of such an inverse identification according to the implementation.

*DMN architecture*

Now the basic architecture of the formulation of DMN and its fitting parameters (**w**, $\theta$) are dicussed.

DMN is a multiple-rank laminate microstructure, comprised of hierarchically nested laminates of laminates on different length scales, or levels.

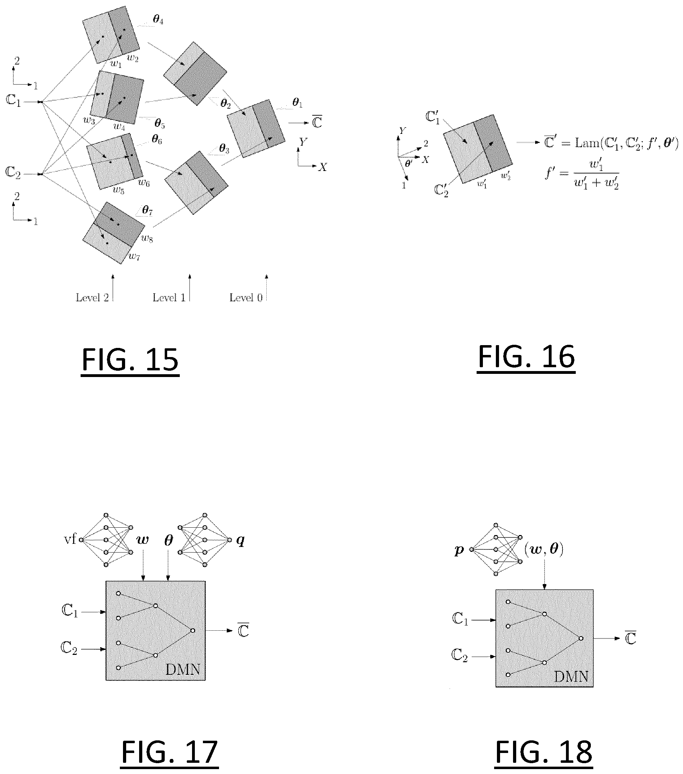

FIG. 15 presents an example of a DMN with three levels (i.e., level 0, level 1, and level 2). The rank n of such multiple-rank laminates, also called the number of DMN layers, characterizes the number of nesting levels. Its architecture corresponds topologically to a perfect binary tree. Each "node" is a rank-1 laminate microstructure, serving as the "mechanistic building blocks" or "neurons" in this neural-network like architecture. FIG. 16 presents an example of one neuron of a DMN which is discussed later in detail.

DMN computes an approximation of the effective stiffness tensor $\overline{\mathbb{C}}$ of the metamaterial, based on the stiffness tensors of its two constituents $\mathbb{C}_1, \mathbb{C}_2$. On the lowest level, the inputs of $\mathbb{C}_1, \mathbb{C}_2$. are fed to the DMN architecture. On the uppermost level (i.e., level 0), the output $\overline{\mathbb{C}}$ is computed.

The stiffness tensors $\mathbb{C}_1, \mathbb{C}_2$ are given in their respective material frames. They can be expressed by several material parameters that describe their elastic behaviors: Young's modulus $E$ (that describes the stiffness required per unit extension in a certain direction), Poisson ratio $v$ (that describes the relative deformation between two orthogonal directions) and shear modulus $\mu$ (that describes the shear stiffness required per unit shear deformation).

For DMN offline training, the implementation sets $\mathbb{C}_1, \mathbb{C}_2$ as orthotropic materials as known in the field, for example according to the already cited Document Liu et al., (2019). A deep material network for multiscale topology learning and accelerated nonlinear modeling of heterogeneous materials. Computer Methods in Applied Mechanics and Engineering, 345, 1138-1168. In this case, the stiffness tensors are expressed by 9 material parameters ($E_1$, $E_2$, $E_3$, $v_{12}$, $v_{13}$, $v_{23}$, $\mu_{12}$, $\mu_{13}$, $\mu_{23}$). The compliance matrix, that is the inverse of the stiffness matrix, is given by the following $6 \times 6$ symmetric matrix:

$$\mathbb{C}^{-1} = \begin{bmatrix} \dfrac{1}{E_1} & -\dfrac{v_{12}}{E_1} & -\dfrac{v_{13}}{E_1} & & & \\ -\dfrac{v_{12}}{E_1} & \dfrac{1}{E_2} & -\dfrac{v_{23}}{E_2} & & & \\ -\dfrac{v_{13}}{E_1} & -\dfrac{v_{23}}{E_2} & \dfrac{1}{E_3} & & & \\ & & & \dfrac{1}{2\mu_{12}} & & \\ & & & & \dfrac{1}{2\mu_{13}} & \\ & & & & & \dfrac{1}{2\mu_{23}} \end{bmatrix}$$

in which a blank elements denotes zero.

*Neuron definition*

The implementation defines each neuron by an analytical function $\overline{\mathbb{C}}' = \mathrm{Lam}(\mathbb{C}'_1, \mathbb{C}'_2, f', \boldsymbol{\theta}')$ that computes the effective homogenized stiffness tensor $\overline{\mathbb{C}}$ ', based on:

- the stiffness tensors $\mathbb{C}'_1, \mathbb{C}'_2$ of two constituents composing the laminate microstructure,
- the volume fraction $f$ of one of the constituent in the laminate microstructure, and
- the rotation $\theta$' of the current neuron with respect to other neurons on upper levels.

The implementation, without the loss of generality, uses the volume fraction of the first constituent.
The exact formula of Lam function depends on the spatial dimension and the physics being considered: mechanical, thermal, etc. Examples of the formula for mechanical behaviors may be found in the already cited document Liu et al., (2019). A deep material network for multiscale topology learning and accelerated nonlinear modeling of heterogeneous materials. Computer Methods in Applied Mechanics and Engineering, 345, 1138-1168. An example is now given below for 2D mechanical behaviors (i.e., plane strain or plane stress conditions) in reference to FIG. 16.
In this example, it is assumed that the interface between two constituents is defined by a plane with a normal vector aligned with the direction 2. Due to mechanical static equilibrium, the stress components along the normal direction $\sigma_{22}$ and $\sigma_{12}$ are continuous across the interface. The strain component parallel to the interface $\varepsilon_{11}$ is also continuous. These interface conditions can be encoded by the following equation involving the strain tensors $\varepsilon = (\varepsilon_{11}, \varepsilon_{22}, \varepsilon_{12})$ and the modified stiffness tensors, denoted by $\widehat{\mathbb{C}}_1$ and $\widehat{\mathbb{C}}_2$ :

$$\begin{bmatrix} 1 & 0 & 0 \\ \mathbb{C}_1[2,1] & \mathbb{C}_1[2,2] & \mathbb{C}_1[2,3] \\ \mathbb{C}_1[3,1] & \mathbb{C}_1[3,2] & \mathbb{C}_1[3,3] \end{bmatrix} \begin{bmatrix} (\boldsymbol{\varepsilon}_1)_{11} \\ (\boldsymbol{\varepsilon}_1)_{22} \\ (\boldsymbol{\varepsilon}_1)_{12} \end{bmatrix} = \begin{bmatrix} 1 & 0 & 0 \\ \mathbb{C}_2[2,1] & \mathbb{C}_2[2,2] & \mathbb{C}_2[2,3] \\ \mathbb{C}_2[3,1] & \mathbb{C}_2[3,2] & \mathbb{C}_2[3,3] \end{bmatrix} \begin{bmatrix} (\boldsymbol{\varepsilon}_2)_{11} \\ (\boldsymbol{\varepsilon}_2)_{22} \\ (\boldsymbol{\varepsilon}_2)_{12} \end{bmatrix}$$

Using the definition of the effective strain tensor $\overline{\varepsilon} = f\varepsilon_1 + (1-f)\varepsilon_2$, where $f$ is the volume fraction of the constituent 1, the following formula that computes the local strain tensor of the constituent 1 from the effective strain tensor can be obtained:

$$\left((1-f)\widehat{\mathbb{C}}_1 + f\widehat{\mathbb{C}}_2\right)\varepsilon_1 = \widehat{\mathbb{C}}_2\overline{\varepsilon} \quad \Rightarrow \quad \varepsilon_1 = \widehat{\mathbb{C}}^{-1}\widehat{\mathbb{C}}_2\overline{\varepsilon}, \qquad \widehat{\mathbb{C}} = (1-f)\widehat{\mathbb{C}}_1 + f\widehat{\mathbb{C}}_2$$

Finally, using the definition of the effective stress tensor $\overline{\sigma} = f\sigma_1 + (1-f)\sigma_2 = f\mathbb{C}_1\varepsilon_1 + (1-f)\mathbb{C}_2\varepsilon_2$, the final formula containing the effective stiffness tensor $\overline{\mathbb{C}}$ that is being sought for:

$$\overline{\sigma} = \overline{\mathbb{C}}\overline{\varepsilon}, \quad \overline{\mathbb{C}} = f(\mathbb{C}_1 - \mathbb{C}_2)\hat{\mathbb{C}}^{-1}\hat{\mathbb{C}}_2 + \mathbb{C}_2$$

The rotation is applied at the end, using the following rotation matrix:

$$R = \begin{bmatrix} \cos^2\theta & \sin^2\theta & \sqrt{2}\sin\theta\cos\theta \\ \sin^2\theta & \cos^2\theta & -\sqrt{2}\sin\theta\cos\theta \\ -\sqrt{2}\sin\theta\cos\theta & \sqrt{2}\sin\theta\cos\theta & \cos^2\theta - \sin^2\theta \end{bmatrix}$$

This yields to $\overline{\mathbb{C}}' = R^T\overline{\mathbb{C}}R$, where $R^T$ is the transpose of the rotation matrix.

In other examples, the implementation can define neurons to predict the effective thermal conductivity of the metamaterial. In this case, $\mathbb{C}_1', \mathbb{C}_2'$ becomes the thermal conductivity tensor of the two constituents, which are 2×2 symmetric tensors in the 2D case. Due to thermal equilibrium equations, the interface equation becomes:

$$\begin{bmatrix} 1 & 0 \\ \mathbb{C}_1[2,1] & \mathbb{C}_1[2,2] \end{bmatrix}\begin{bmatrix} (\nabla T_1)_1 \\ (\nabla T_1)_2 \end{bmatrix} = \begin{bmatrix} 1 & 0 \\ \mathbb{C}_2[2,1] & \mathbb{C}_2[2,2] \end{bmatrix}\begin{bmatrix} (\nabla T_2)_1 \\ (\nabla T_2)_2 \end{bmatrix}$$

In the equation, the strain tensor is now replaced by the temperature gradient vector. The final formula containing the effective thermal conductivity tensor $\overline{\mathbb{C}}$ is formally the same as before of type:

$$\overline{q} = \overline{\mathbb{C}}\overline{\nabla T}, \quad \overline{\mathbb{C}} = f(\mathbb{C}_1 - \mathbb{C}_2)\hat{\mathbb{C}}^{-1}\hat{\mathbb{C}}_2 + \mathbb{C}_2.$$

The rotation matrix becomes:

$$R = \begin{bmatrix} \cos\theta & -\sin\theta \\ \sin\theta & \cos\theta \end{bmatrix}.$$

*DMN fitting parameters*

The fitting parameters of DMN are weights $w$, and rotations $\theta$. The weights are defined on the leaf nodes and characterize the respective volume fraction of each material node. They are required to be non-negative. Rotations are defined for each node. They may be described respectively by quaternions.

In the original DMN formulation, these fitting parameters $(w, \theta)$ are supposed to be fixed. Hence this is not adequate for parametric microstructures $p$, for which they should also vary with the microstructural parameters. The implementation thereby accounts for the dependence of $(w, \theta)$ on $p$.

*Physics-Informed Neural Network for DMN fitting parameters*

In the implementation, the functional dependence of DMN parameters $(w, \theta)$ on the microstructural parameters $p$ is directly accounted for by feedforward neural networks composed of multiple layers of affine transformations and activation functions. Due to the physics-based architecture of DMN, the architecture of the hidden layers does not significantly change the performance.

In an option, represented in FIG. 17, the implementation uses a physics-informed architecture in which the DMN weights vector $w$ is solely dependent on the volume fraction parameter, while the DMN rotation vector θ varies with all other parameters that do not change the volume fraction. Hence, the microstructural parameters are partitioned as:

$$p = (vf, q) \in \mathbb{R} \times \mathbb{R}^q,$$

where $vf$ is the volume fraction of the second phase, while $\boldsymbol{q}$ denotes all other $\boldsymbol{q}$ independent parameters that are orthogonal to $vf$. Given this partition, the physics-informed neural network (PINN) for DMN parameters is now given by:

$$\boldsymbol{w(p)} \quad = \boldsymbol{w}(vf) = \boldsymbol{\sigma}(vf \cdot \boldsymbol{w_1} + \boldsymbol{w_0}),$$

$$\boldsymbol{\theta(p)} \quad = \boldsymbol{\theta(q)} = \boldsymbol{\Theta_1 q} + \boldsymbol{\theta_0},$$

where $\boldsymbol{w_0}$ and $\boldsymbol{w_1}$ are vectors of length $n$, $\theta_0$ is a $(2^n - 1) \times 4$ matrix and $\Theta_1$ is a $(2^n - 1) \times 4 \times q$ tensor. These tensors ($\boldsymbol{w_0}$, $\boldsymbol{w_1}$, $\theta_0$, $\Theta_1$) are the fitting parameters of the new proposed architecture. The architecture used in this option is hereinafter called PINN-DMN. In the notation hereinbelow $vf$ (which is a scalar) can be considered as a function of DMN weights $\boldsymbol{w}$ (which can be presented as a vector), as the notation $vf(\boldsymbol{w})$.

In another option, represented in FIG. 18, the implementation used a fully connected architecture, in which the DMN fitting parameters $\boldsymbol{w}$ and $\theta$ depend both on the total parameter vector $\boldsymbol{p}$.

The PINN architecture compared to the fully connected one needs fewer fitting parameters due to the partition. This increases computational efficiency. Furthermore, the numerical simulations suggest that the PINN architecture provides a comparable accuracy compared to the fully connected one and may also enhance generalization ability (interpolation and extrapolation) in the parametric space. This means that the proposed PINN approach maintains high accuracy in the predicted effective properties while varying the microstructural parameters.

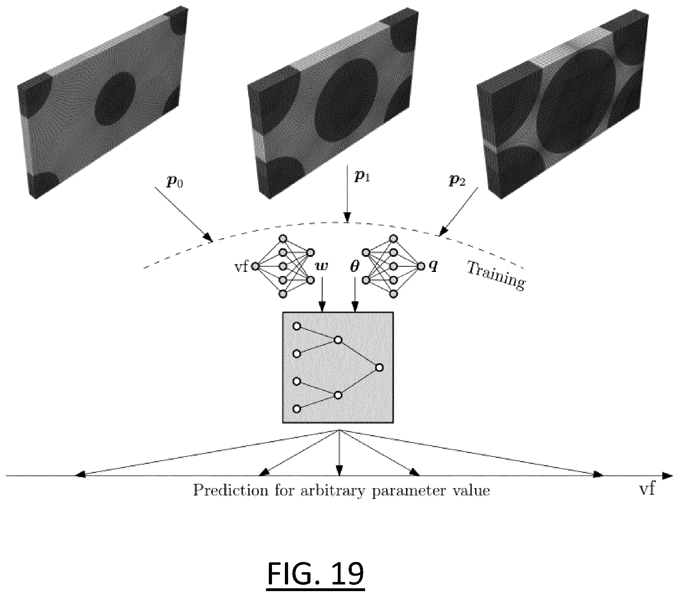

FIG. 19 shows an application example of PINN approach in FIG. 17, in which the method is used to perform the prediction for different value of the parameter, $p_0$, $p_1$, $p_2$.

Compared to the transfer learning approach, a unique offline training is now required to optimize the fitting parameters of PINN-DMN. The neural network is now evaluated jointly using the linear elastic behavior data at each $p_i$ in the parametric space. Furthermore, a neural-network functional dependence naturally defines interpolation and extrapolation inside or outside the training domain and extends easily to higher parametric dimensions.

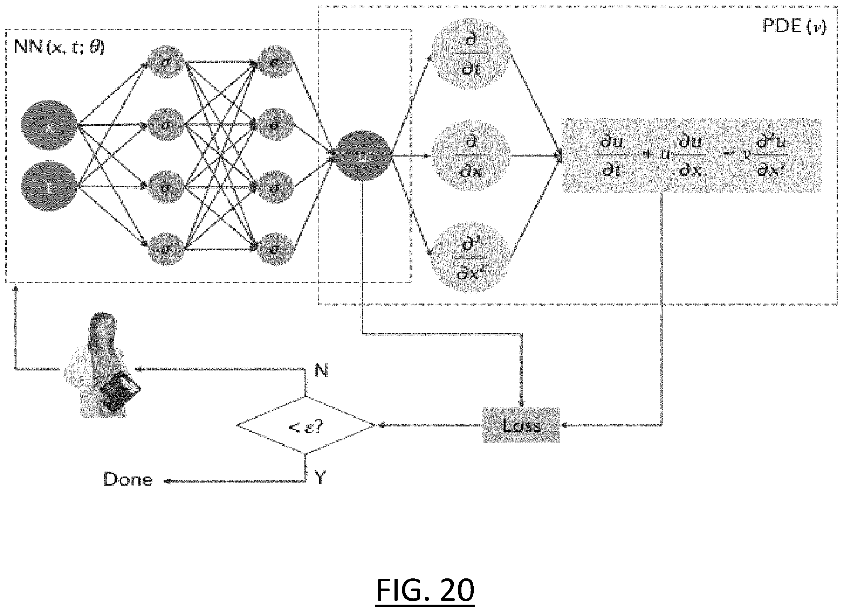

Apart from the physics-informed architecture on the functional dependence $\boldsymbol{p} \mapsto (\boldsymbol{w}, \theta)$, some physical constraints can also be prescribed to further improve its generalization ability, similar to the physics-informed machine learning approach for nonlinear partial differential equations. This so-called physics-informed approach is composed of a standard feedforward neural network and a loss function that accounts for the physics equations or constraints defined by partial differential equations (PDE). An example of such physics-informed network is presented in FIG. 20 where the PDE equations are prescribed as an additional term in the loss function.

In the implementation, two additional physical constraints are prescribed on the neural network that computes $\boldsymbol{p} \mapsto (\boldsymbol{w}, \theta)$. First constraint is a volume fraction constraint $\mathcal{L}_{vf}$. In other words, the DMN volume fraction should match the real volume fraction value of the microstructure for arbitrary volume fraction values. Second constraint is an orientation constraint $\mathcal{L}_a$ which imposes that the average material frame orientation predicted by DMN should also match that of the real microstructure.

Each of the two constraint is discussed now.

*Volume fraction constraint*

The DMN volume fraction can be computed from the DMN weights vector using the following formula:

$$vf(\boldsymbol{w}) = \frac{\sum_{i \in \mathbb{I}_2} \boldsymbol{w_i}}{\sum_{i \in \mathbb{I}} \boldsymbol{w_i}} \approx vf_\Omega$$

In this formula, the $\boldsymbol{w_i}$ are the weights defined on each leaf node (material node) of the DMN architecture. They are divided into two parts: those defined for the constituent 1 with the index set $\mathbb{I}_1$ and those defined for the constituent 2 with $\mathbb{I}_2$. The union of these two index sets is $\mathbb{I}$. The formula above computes the volume fraction of the phase 2 in the whole microstructure. Thanks to the physics-based design of DMN, the real volume fraction of the actual metamaterial $vf_\Omega$ can be well approximated by DMN volume fraction.

Using the PINN-DMN framework, now these DMN weights $\boldsymbol{w_i}$ are computed by a neural network. The implementation may hence prescribe the following volume fraction constraint at all volume fraction values:

$$vf(\boldsymbol{w}) = vf\big(\boldsymbol{\sigma}(vf \cdot \boldsymbol{w_1} + \boldsymbol{w_0})\big) = \boldsymbol{vf}, \quad \forall vf \in [\boldsymbol{0,1}].$$

This formula means the DMN volume fraction always matches the actual volume fraction of the microstructure. In order to reinforce this constraint during offline training, a loss function based on this volume fraction constraint is defined as the following:

$$\mathcal{L}_{vf} = \frac{1}{n}\sum_{i=1}^{n}\big(vf\big(\boldsymbol{\sigma}(vf_i \cdot \boldsymbol{w_1} + \boldsymbol{w_0})\big) - vf_i\big)^2,$$

where $vf_i$ denotes the collocation points to weakly prescribe the volume fraction constraints and n is the number of such collocation points. Since the volume fraction is a scalar between 0 and 1, a uniform sampling is adopted.

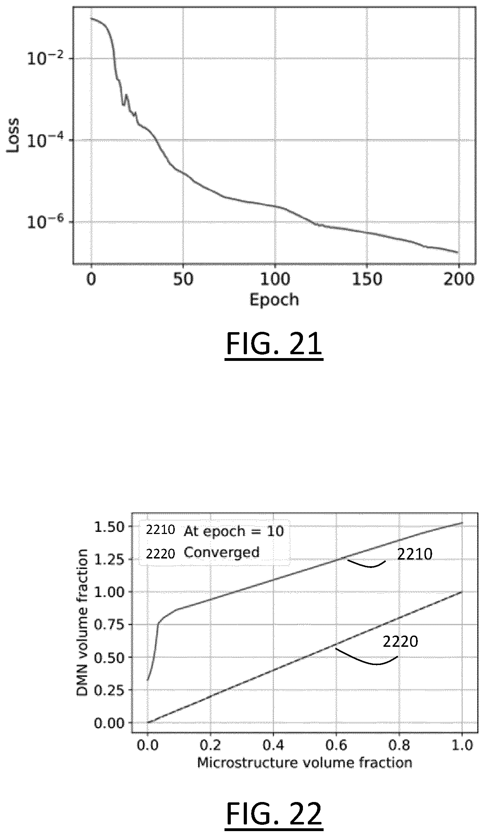

FIG.s 21 and 22 present examples of the volume fraction constraint prescribed on the PINN. FIG. 21 presents the minimization of the volume fraction constraint loss function while FIG. 22 presents the predicted DMN volume fraction at the early stage (i.e., epoch=10) and at the end of training (i.e., converged).

With the decrease of the loss function during the optimization iterations, the DMN volume fraction matches better and better the actual volume fraction of the microstructure.

*Orientation constraint*

DMN not only captures the microstructure morphology. It also learns material orientation distribution function in the microstructure. Using orientation tensors, the implementation proposes an orientation constraint to be prescribed on DMN weights $\boldsymbol{w}$ and rotations $\theta$, in order that the parametric DMN generalizes such material orientation knowledge in the whole parametric space.

Orientation tensors describe concisely the statistical information of the orientation distribution of *unit vectors.* Given an orientation distribution function $f\colon \mathbb{S}^2 \to \mathbb{R}$, where $\mathbb{S}^2$ denotes the two-dimensional surface of a unit sphere, the $3 \times 3$ second-order orientation tensor is defined by:

$$\boldsymbol{a} = \int_{\mathbb{S}^2} f(\boldsymbol{e})\boldsymbol{e} \otimes \boldsymbol{e}\, d\boldsymbol{e}, \quad \|\boldsymbol{e}\| = 1.$$

It can be easily shown that a is symmetric and $tr(\boldsymbol{a}) = 1$, due to the normalization constraints of $e$ and of the probability density $f$. Higher order orientation tensors do exist, however the second order one is the most frequently used to characterize local fiber orientations due to manufacturing processes and their influence on material properties.

The implementation uses a generalization of such (second order) orientation tensors for orientation distributions of rotations. Contrary to transversely isotropic fibers for which a single unit vector suffices to characterize its material frame, general anisotropic materials (like orthotropic ones) require a rotation matrix $\boldsymbol{R}$ to describe the transformation from its material frame ($\boldsymbol{e}_1$, $\boldsymbol{e}_2$, $\boldsymbol{e}_3$) to the global one. In such cases, the orientation distribution function is now defined for rotation matrices $f\colon SO(3) \to \mathbb{R}$. The domain of this function, $SO(3)$, is the 3D rotation group, containing all rotation matrices in the three-dimensional space that are orthogonal with unit determinant. Given that each column $1 \leq i \leq 3$ of $\boldsymbol{R}$ essentially expresses $\boldsymbol{e}_i \in \mathbb{S}^2$ in the global frame, it defines an orientation distribution of the material frame.

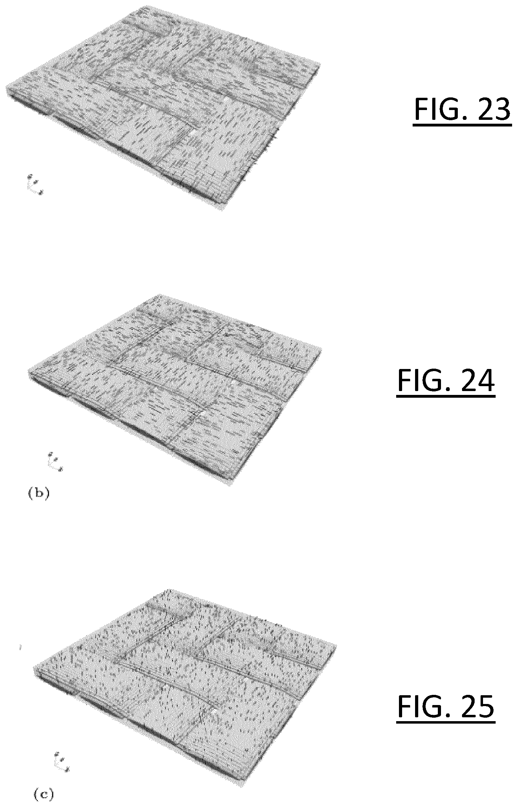

FIG.s 23-25 present examples of such orientation distributions for each of the three material axes provided for the tows in the woven microstructure. FIG. 23 is for the direction $\boldsymbol{e}_1$, FIG. 24 is for the direction $\boldsymbol{e}_2$, and FIG. 25 is for the direction $\boldsymbol{e}_3$.

Given this interpretation, three (second order) orientation tensors can be defined for each axis of the material frame:

$$a^{(i)} = \int_{\mathbb{S}^2} f(\boldsymbol{R})\boldsymbol{e}_i \otimes \boldsymbol{e}_i\, d\boldsymbol{e}, \quad \|\boldsymbol{e}_i\| = 1,$$

where Einstein summation is not implied. The tensor a can be understood as a third-order material frame orientation tensor. For discrete probability functions defined on a mesh, the integral can be understood as weighted-averaging using the element volumes as weights. Note that in the case of a two-phase microstructure, it can be computed for each of the phase.

In the example above, the material frame orientation tensor for the tows is given by:

$$a^{(1)} = \begin{bmatrix} 0.5 & 0 & 0 \\ 0 & 0.5 & 0 \\ 0 & 0 & 0 \end{bmatrix}, \quad a^{(2)} = \begin{bmatrix} 0.5 & 0 & 0 \\ 0 & 0.5 & 0 \\ 0 & 0 & 0 \end{bmatrix}, \quad a^{(3)} = \begin{bmatrix} 0 & 0 & 0 \\ 0 & 0 & 0 \\ 0 & 0 & 1 \end{bmatrix}.$$

The tows are thus isotropically oriented in the X-Y plane for $e_1$ and $e_2$, and unidirectionally oriented in the Z axis for $e_3$. For the matrix phase, its material frame coincides with the global frame. In this case, a is similar to unidirectional orientation tensors and satisfies:

$$a_{ii}^{(i)} = 1, \quad a_{jk}^{(i)} = 0 \quad \textit{for other components.}$$

Such orientation tensors can also be defined for DMN. DMN rotations $\theta$ define rotation matrices between the local frame of the current laminate to that of the next nesting level. They can thus be composed to obtain the effective rotation matrix from the material frame $(e_1, e_2, e_3)$ (leaf laminates) to the global frame of the microstructure $\Omega$ (root laminate). Let $p$ (i) denote the parent of a laminate $i$ in the DMN binary tree architecture. For instance, in the 3-layer DMN example shown in FIG. 15, $p(4) = 2$ for the leaf laminate 4 and $p^2(4) = p(p(4)) = p(2) = 1$ which is the root laminate. For each leaf laminate $i$ which carries the DMN material nodes, the effective rotation matrix from the material frame to the global one is given by:

$$\widetilde{R}_i = R\left(\theta_{p^{n-1}(i)}\right) \cdots R\left(\theta_{p^2(i)}\right) R\left(\theta_{p(i)}\right) R(\theta_i), \quad \widetilde{R}_i \in SO(3).$$

Note that for an n-layer DMN, it holds that $p^{n-1}(i) = 1$ for arbitrary leaf laminate i. Due to the absence of input rotation matrices for $(\mathbb{C}_1, \mathbb{C}_2)$, material nodes that share the same leaf laminate also obtain the same effective rotation matrix. For instance, for the material nodes 3 and 4 contained in the leaf laminate 5, their effective rotation is:

$$\widetilde{R}_5 = R(\theta_1)R(\theta_2)R(\theta_5).$$

Using these effective rotations on leaf laminates, the DMN material frame orientation tensor can be computed for the phase 1 as the following:

$$a_{DMN}^{(i)}(\boldsymbol{w}, \boldsymbol{\theta}) = \frac{\sum_{i \in \mathbb{I}_1} w_i \, \tilde{e}_i \otimes \tilde{e}_i}{\sum_{i \in \mathbb{I}_1} w_i}.$$

Similar formula can be defined for the phase 2. Compared to the DMN volume fraction, the computation of DMN orientation tensors requires both DMN weights $\boldsymbol{w}$ and rotations $\theta$. Using the physics-informed neural network for DMN parameters, the implementation exploits the following orientation constraint through the definition of a loss function:

$$\mathcal{L}_a = \frac{1}{n} \sum_{i=1}^{n} \| a_{DMN}\left(\boldsymbol{w}(\boldsymbol{p}_i), \boldsymbol{\theta}(\boldsymbol{p}_i)\right) - a(\boldsymbol{p}_i) \|^2, \quad \| a \|^2 = \sum_{i=1}^{3}\sum_{j=1}^{3}\sum_{k=1}^{3}\left(a_{jk}^{(i)}\right)^2.$$

Similar loss function is defined for the phase 2 and then summed together. In this equation, $\boldsymbol{p}_i$ are the collocation points in the microstructural parametric space and $a(\boldsymbol{p}_i)$ are the actual orientation tensors of the parametric microstructure. Similar to the volume fraction constraint, the number $n$ denotes the number of these collocation points. These collocation points can be sampled using the Latin hypercube sampling method.

*Material sampling*

The implementation follows the original material sampling method proposed by the already cited document Liu et al., (2019). A deep material network for multiscale topology learning and accelerated nonlinear modeling of heterogeneous materials. Computer Methods in Applied Mechanics and Engineering, 345, 1138-116. The implementation assumes that $(\mathbb{C}_1, \mathbb{C}_2)$ are both orthotropic in their respective material frames. In total, 9 + 9 = 18 material parameters are required to characterize their orthotropic elastic behaviors and 1 additional scaling parameter is used to introduce contrasts in the elastic moduli between the two phases. In this work, Latin hypercube sampling is used to sample this 19-dimensional space.

**Total loss function**

These two constraints are added to the total loss function, based on the Mean Squared Error between the DMN predicted homogenized elasticity tensors and those generated by FE-RVE simulations. These errors among material samples $\mathbb{M}$ are then averaged to compute the loss $\mathcal{L}_p$ at a fixed microstructural parameter value. Finally, they are averaged to the total loss function, among samples $\mathbb{P}$ in the parametric space.

$$\mathcal{L} = \frac{1}{|\mathbb{P}|} \sum_{\mathbb{P}} \mathcal{L}_p + \mathcal{L}_{vf} + \mathcal{L}_a, \qquad \mathcal{L}_p = \frac{1}{|\mathbb{M}|} \sum_{\mathbb{M}} e_i^2, \qquad e_i = \frac{\| \overline{\mathbb{C}_i}^{DMN} - \overline{\mathbb{C}_i}^{FE} \|}{\| \overline{\mathbb{C}_i}^{FE} \|}$$

The total loss function is minimized using gradient-based methods. In the implementation, the PINN-DMN architecture is implemented using PyTorch. The derivatives of the loss function with respect to the fitting parameters can thus be easily computed with automatic differentiation.

*Example on a specific parametric microstructure*

The implementation was tested on a specific parametric microstructure: the 2 × 2 twill woven composites with varying tow volume fractions. The objective is to evaluate PINN-DMN for such microstructures in terms of elastic moduli prediction as a function of the volume fraction. Inverse identification of the material and microstructural parameters is also considered using PINN-DMN as an accurate and efficient surrogate of the parametric microstructure.

The 3D finite element model was constructed using TexGen with 4 volume fraction values for the tows. Three of them (*vf* = 0.459, *vf* = 0.608 and *vf* = 0.729) were used to generate training dataset, while *vf* = 0.537 was used to test interpolation accuracy. The FE-RVE analysis was conducted under Abaqus to obtain the 6 × 6 linear elastic stiffness tensor. Each FE-RVE model contains 75000 voxel elements and 241899 degrees of freedom. The simulation was performed with 24 threads using Intel(R) Xeon(R) Gold 5220R CPU @2.20GHz and requires approximately 11 seconds.

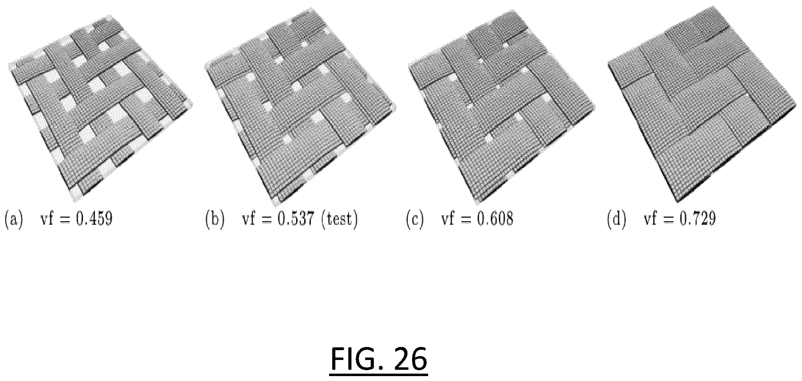

FIG. 26(a)-(d) presents FE-RVE models for the woven composite with different fiber volume fractions. FIG. 26(a) is for *vf* = 0.459, FIG. 26(b) for is *vf* = 0.608, FIG. 26(c) is for *vf* = 0.729, and FIG. 26(d) for *vf* = 0.537. The first three models are used to generate training dataset, while the last one is used to test interpolation accuracy.

The real linear elastic properties of the two phases can be found below. The matrix is isotropic while the carbon fiber tows are assumed to be transversely isotropic in the local material frames. For training microstructures *vf* = 0.459, *vf* = 0.608 and *vf* = 0.729, 400 of the 500 material samples are used as training dataset, while the other 100 are reserved for validation. For *vf* = 0.537, all the 500 samples are used for testing the interpolation accuracy.

*Table 1 Real linear elastic properties of the two phases for the woven composite.*

|  | *E* (MPa) | *v* |
|---|---|---|
| Matrix | 3800 | 0.387 |

*Table 2 Real linear elastic properties of the two phases for the woven composite*

|  | $E_1$ (GPa) | $E_2$ (GPa) | $v_{12}$ | $v_{23}$ | $\mu_{12}$ (GPa) |
|---|---|---|---|---|---|
| Tow | 78.8 | 6.24 | 0.35 | 0.6 | 2.39 |

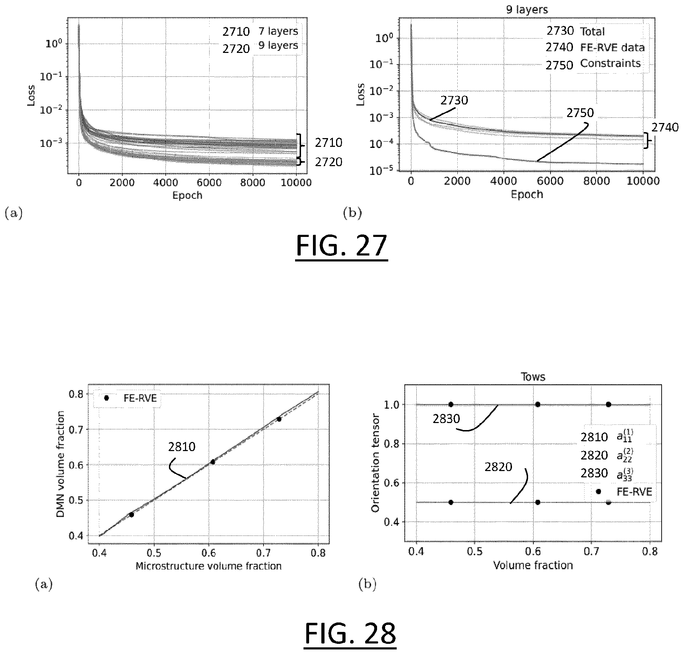

PINN-DMN was trained following with the two physical constraints. The loss histories were compared for 7 and 9 DMN layers. The neural network becomes more accurate with increasing layers, leading to smaller (converged) loss values. The total loss can also be partitioned to a FE-RVE data part and a physical constraints part. These two parts are monotonically decreasing. The physical constraints are well satisfied since the corresponding loss value is approaching $10^{-5}$ with 9 layers. In the sequel, the results using 7 layers are reported since it provides satisfactory accuracy. FIG. 27 shows PINN-DMN training for the woven composites. While FIG. 27(a) shows loss histories with 7 and 9 DMN layers, FIG. 27(b) shows partition of the total loss into the FE-RVE data part and the physical constraints part.

The volume fraction and the material frame orientation tensor for the tows are compared with their prescribed values. The DMN volume fraction matches the FE-RVE data points and agrees well with the theoretical straight line even when evaluated outside the training region. The components $a_{ii}^{(i)}$ of the DMN orientation tensor are shown below. An excellent agreement is also obtained.

FIG. 28 shows the verification of the physical constraints for the woven composite. FIG. 28(a) is the volume fraction and FIG. 28(b) is material frame orientation tensor for the tows.

The training, validation and test errors are computed at different volume fractions. The median error is approximately 2% for all the four microstructures. The test error is only slightly larger compared to the training ones.

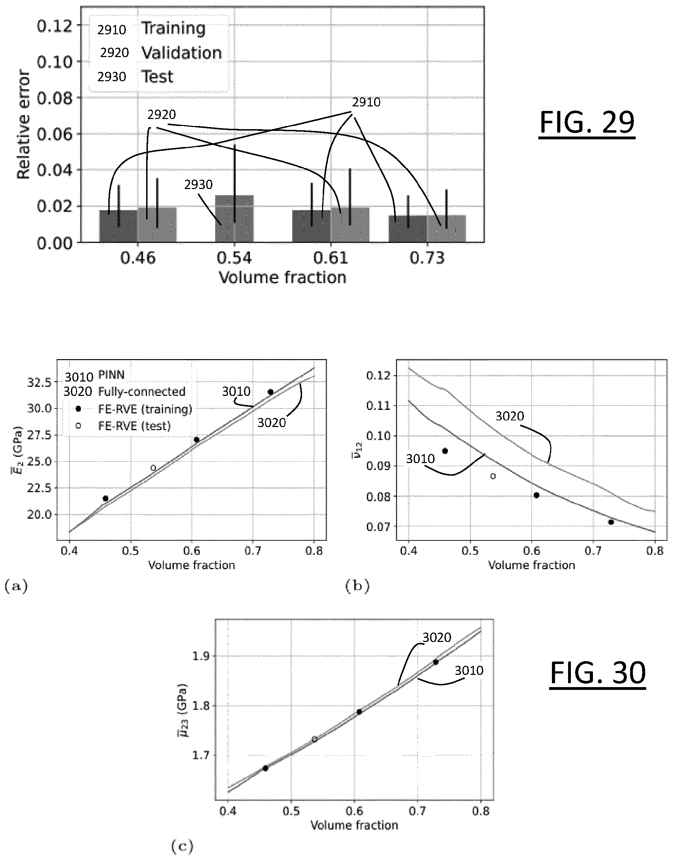

FIG. 29 shows training, validation, and test errors of PINN-DMN at different volume fractions represented by their respective 0.1, 0.5 (median) and 0.9-quantiles.

With the real linear elastic properties, the homogenized elastic moduli are computed with varying volume fraction. For comparison, the fully connected architecture is also tested with the physical constraints. Recall that the fully connected architecture implies that the DMN rotations also become a function of the volume fraction. Both models capture well the nonlinear *vf*-dependence of these elastic moduli. The fully connected architecture not only increases the number of fitting parameters ($\Theta_1$ is now added), but it does also not improve prediction accuracy compared to PINN.

FIG.30 shows parametric DMN models: FIG.30(a) is in-plane Young's modulus $\overline{E}_2$, FIG.30(b) is transverse Poisson ratio $\overline{\nu}_{12}$, and FIG.30(c) is in-plane shear modulus $\overline{\mu}_{23}$. The FE-RVE training and test data are indicated.

The computational efficiency of DMN in terms of computing the homogenized elasticity tensor given $\mathbb{C}_1$ and $\mathbb{C}_2$ is compared to FE-RVE simulations. Thanks to this significant speed-up, DMN can be further used for parametric analysis, uncertainty quantification and material property calibration. The following table represents computational speed-up of DMN compared to FE-RVE in terms of homogenized elasticity tensor prediction. The experiments have been performed on 24 cores of Intel(R) Xeon(R) Gold 5220R CPU @ 2.20GHz for both cases.

*Table 3 Computational speed-up of DMN compared to FE-RVE*

|  | FE-RVE | 7-layer DMN |
|---|---|---|
| Wall time (speed-up) | 11s | 6.62 ms (1662) |

*Inverse identification of input material and microstructural parameters*

Trained PINN-DMN can serve as an accurate and computationally efficient surrogate of the parametric microstructure. Not only it can be used in forward prediction of effective properties at different microstructural parameters, but it can also be employed to identify both the *material* and *microstructural* properties in an inverse identification problem.

In this application, the homogenized elasticity tensor $\overline{\mathbb{C}}$ is provided, and the objective is to identify the elastic properties of the matrix $\mathbb{C}_1$, those of the tows $\mathbb{C}_2$ as well as the volume fraction of the tows *vf*. In practice $\overline{\mathbb{C}}$ can be measured experimentally. Here the implementation uses the FE-RVE simulation result $\overline{\mathbb{C}}$ on the test microstructure *vf* = 0.537, obtained with the real properties which are now sought for.

Motivated by the fact that the gradients with respect to $(\mathbb{C}_1, \mathbb{C}_2, vf)$ can be easily computed using automatic differentiation thanks to the PINN-DMN architecture, the implementation adopts a gradient-based optimization approach based on a loss function which compares DMN prediction and the input homogenized data $\overline{\mathbb{C}}_{\text{Data}}$ with the standard Frobenius norm.

$$\mathcal{L}_{cal} = \frac{\parallel \overline{\mathbb{C}}_{DMN} - \overline{\mathbb{C}}_{Data} \parallel^2}{\parallel \overline{\mathbb{C}}_{Data} \parallel^2}.$$

The optimization iterations require the initial guess values for the unknowns $(\mathbb{C}_1, \mathbb{C}_2, vf)$. Hence, the real properties as well as the true volume fraction $vf = 0.537$ are randomly perturbed using a normal distribution with a coefficient of variation equal to 20%. The initial values generated by two random realizations are indicated in the two tables below.

*Table 4 Initial linear elastic properties of the two phases for the inverse identification problem.*

| Matrix | $E$ (MPa) | $v$ |
|---|---|---|
| Realization 1 | 4491 | 0.400 |
| Realization 2 | 3908 | 0.437 |

*Table 5 Initial linear elastic properties of the two phases for the inverse identification problem.*

| Tow | $vf$ | $E_1$ (GPa) | $E_2$ (GPa) | $v_{12}$ | $v_{23}$ | $\mu_{12}$ (GPa) |
|---|---|---|---|---|---|---|
| Realization 1 | 0.550 | 92.3 | 5.60 | 0.347 | 0.697 | 2.14 |
| Realization 2 | 0.541 | 65.4 | 5.22 | 0.297 | 0.764 | 1.78 |

The loss histories corresponding to these two initial values are given. The loss function converges quickly and may reach $10^{-5}$ within 1000 optimization iterations. The input homogenized elasticity tensor $\overline{\mathbb{C}}$ is well recovered. The relative error between the converged homogenized elasticity tensor and the input $\overline{\mathbb{C}}$ data is 0.42% and 0.26% for these two sets of initial values.

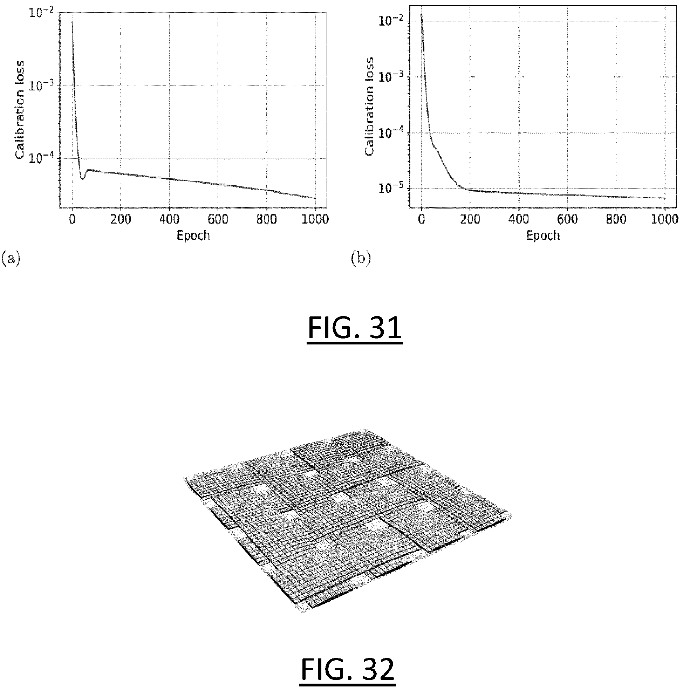

FIG. 31 shows the calibration loss histories using two initial values: (a) realization 1; (b) realization 2.

*Table 6 Converged homogenized elasticity tensor from two different realizations of initial values, compared with the true data.*

| $\overline{\mathbb{C}}$ | $E_1 = E_2$ (GPa) | $E_3$ (GPa) | $v_{12}$ | $v_{13} = v_{23}$ | $\mu_{12}$ (GPa) | $\mu_{13} = \mu_{23}$ (GPa) | Error |
|---|---|---|---|---|---|---|---|
| Data | 24.4 | 7.03 | 0.0866 | 0.558 | 1.87 | 1.73 | |
| Realization 1 | 24.5, 24.3 | 7.05 | 0.0879 | 0.556, 0.555 | 1.87 | 1.73 | 0.42% |
| Realization 2 | 24.4 | 7.02 | 0.0863 | 0.560, 0.558 | 1.86 | 1.74 | 0.26% |

The inversely identified input parameters $(\mathbb{C}_1, \mathbb{C}_2, vf)$ are reported. The material and microstructural parameters found with the realization 1 are similar to the actual properties. However, with the realization 2, the identified volume fraction 0.667 is higher than the data 0.537. Meanwhile, the longitudinal Young's modulus (64.6 GPa) of the tows is also smaller than the data (78.8 GPa). This illustrates the non-uniqueness of the inverse identification problem. Additional conditions may be provided by the user to further constrain the inverse problem. For instance, some input properties, like those of the matrix, can be assumed to be fixed. Nevertheless, such inverse identification problems can now be solved efficiently using PINN-DMN within seconds.

*Table 7 Identified linear elastic properties of the two phases and the volume fraction for the inverse identification problem.*

| Matrix | $E$ (MPa) | $v$ |
|---|---|---|
| Realization 1 | 4309 | 0.385 |
| Realization 2 | 4624 | 0.375 |

*Table 8 Identified linear elastic properties of the two phases and the volume fraction for the inverse identification problem.*

| Tow | *vf* | $E_1$ (GPa) | $E_2$ (GPa) | $\nu_{12}$ | $\nu_{23}$ | $\mu_{12}$ (GPa) |
|---|---|---|---|---|---|---|
| Realization 1 | 0.533 | 80.7 | 5.53 | 0.340 | 0.614 | 2.09 |
| Realization 2 | 0.667 | 64.6 | 5.27 | 0.364 | 0.617 | 1.90 |

*Compressed representation of microstructures with arbitrary parameters*

Traditionally, parametric microstructures can be described computationally using unstructured meshes that discretize spatially the microstructure. For the woven microstructure for instance, a typical mesh like FIG. 32 may be provided with a fixed volume fraction value. For a different parameter value, another mesh is to be provided.

For each mesh, it requires the storage of the nodes coordinates and the cell connectivity matrix. In this case, it requires approximately 4 MB. Note that it only concerns one particular volume fraction value. If five volume fractions are required, then five microstructure meshes need to be stored. The mesh-based representation may become resource demanding when many parameter values are required.

*Table 9*

| Mesh-based representation | Nodes | Cells | Total bytes |
|---|---|---|---|
| Data structure | 80000 × 3 reals | 75000 × 8 integers | $4.32 \times 10^6$ |

The parametric DMN architecture of the implementation serves also as a new compressed representation of parametric microstructures. Contrary to a mesh-based representation, the parametric DMN approach describes the whole parametric microstructure. With a 7-layer parametric DMN architecture, only 6 KB is required to represent the parametric microstructure with arbitrary parameter value variation.

*Table 10*

| The implementation | Weights | Rotations | Total bytes |
|---|---|---|---|
| Data structure | $2^8$ = 256 reals | $4 \times (2^7 - 1)$ = 508 reals | $6.112 \times 10^3$ |

This compressed representation can be used to construct a database of parametric multi-scale materials. Each entry corresponds to a particular parametric multi-scale material with corresponding morphological parameters that are allowed to vary. Only the optimized neural network parameters are needed to be stored. The below table presents a tabular example of such entries.

*Table 11*

| Multiscale material | Morphological parameters | Optimized parameters |
|---|---|---|
| Unidirectional fiber composite | Volume fraction, fiber misalignment | $(\boldsymbol{w}_0, \boldsymbol{w}_1, \theta_0, \Theta_1)$ = (0.1, ...) |
| Woven composite | Volume fraction | $(\boldsymbol{w}_0, \boldsymbol{w}_1, \theta_0, \Theta_1)$ = (0.2, ...) |
| Short fiber-reinforced plastics | Volume fraction, aspect ratio, fiber orientation | $(\boldsymbol{w}_0, \boldsymbol{w}_1, \theta_0, \Theta_1)$ = (-0.8, ...) |
| ... | ... | ... |

**Claims**

1. A computer-implemented method for training a Deep Material Network (DMN)-based neural network configured to predict a macroscopical physical property of a multi-scale material, the multi-scale material comprising one or more components, the method comprising:

   - obtaining a dataset, each entry of the dataset corresponding to a respective multi-scale material object, the entry comprising:

◦ a tensor ( $\overline{\mathbb{C}}$ ) describing the physical property of the multi-scale material object at a macroscopical level,

◦ one or more tensors $(\mathbb{C}_1, \mathbb{C}_2)$ each describing the physical property of a component of the multi-scale material object at a microscopical level, and

◦ one or more morphological parameters ($p$) each describing a morphology of the multi-scale material object; and

- training, based on the obtained dataset, the neural network to predict a tensor describing the physical property of a multi-scale material object at a macroscopical level based on the one or more tensors ( $(\mathbb{C}_1, \mathbb{C}_2)$ ) for the object and based on the one or more morphological parameters for the object.

2. The method of claim 1, wherein the Deep Material Network (DMN)-based neural network consists of a first block and a second block; wherein

- the first block is configured to receive as input the one or more morphological parameters ($p$) for the object and to output a value of a plurality of network parameters (($w, \theta$)), and
- the second block is configured to receive as input the value of the plurality of network parameters (($w, \theta$)) and the one or more tensors for the object ( $(\mathbb{C}_1, \mathbb{C}_2)$ ), and to output a prediction of the physical property of the multi-scale material object at a macroscopical level.

3. The method of claim 2, wherein:

- the first block is a feed-forward neural network; and/or
- the second block has a DMN architecture with the plurality of network parameters (($w, \theta$)).

4. The method of any of claims 2 to 3, wherein the first block is a fully connected neural network.

5. The method of any of claims 2 to 3, wherein the first block consists of a first sub-block and a second sub-block; wherein:

- the first sub-block is configured to a receive as input a value of a first subset ($vf$) of the one or more morphological parameters ($p$) and to output a value of a respective subset ($w$) of the plurality of network parameters (($w, \theta$)), and
- the second sub-block is configured to a receive as input a value of second subset ($q$) of the one or more morphological parameters ($p$) and to output a value of a respective subset ($\theta$) of the plurality of network parameters (($w, \theta$)).

6. The method of claim 5, wherein:

- the first subset of the one or more morphological parameters comprises a set of volume fraction for each of the components; and/or
- the second subset of the one or more morphological parameters comprises one or more orientation parameters.

7. The method of any of claims 1 to 6, wherein the training comprises minimizing a loss function, the loss function penalizing, for each entry, a disparity between:

- the obtained tensor describing the physical property of the multi-scale material object at a macroscopical level, and
- the predicted tensor describing the physical property of the multi-scale material object at a macroscopical level.

8. The method of claim 7, wherein the loss function further penalizes a non-respect of a volume fraction constraint for the multi-scale material object and/or a non-respect of a material orientation constraint for the multi-scale material object.

9. A neural network trainable according to any of preceding claims.

10. A computer-implemented method of use of the neural network of claim 9, the method comprising:

- obtaining:

◦ one or more tensors each describing the physical property of a component of the multi-scale material object at a microscopical level, and

◦ one or more morphological parameters each describing a morphology of the multi-scale material object; and

- predicting the macroscopical physical property of the multi-scale material object by applying the neural network on the one or more tensors and the one or more parameters,

and/or

- obtaining:

◦ a tensor ( $\overline{\mathbb{C}}$ ) describing the physical property of the multi-scale material object at a macroscopical level, and

- determining:

◦ one or more tensors each describing the physical property of a component of the multi-scale material object at a microscopical level, and

◦ one or more morphological parameters each describing a morphology of the multi-scale material object;

wherein the determining comprises minimizing a disparity between the obtained tensor and a candidate predicted value for the obtained tensor, the candidate predicted value being determined by applying the neural network on candidate one or more tensors and candidate one or more morphological parameters.

11. The method of claim 10, wherein the minimizing of the disparity comprises computing:

- a gradient of the candidate predicted value with respect to each respective candidate one or more tensors, and
- a gradient of candidate predicted value with respect to each respective candidate one or more morphological parameters.

12. A database of multi-scale materials, each entry of the dataset corresponding to a respective multi-scale material, the entry comprising:

- one or more morphological parameters each describing a morphology of the multi-scale material object, and
- a neural network trained for predicting a macroscopical physical property of a multi-scale material according to any of claims 1 to 8.

13. A computer program comprising instructions for performing the method according to any one of claims 1 to 8 and/or 10 to 11.

14. A computer readable storage medium having recorded thereon the computer program of claim 13 and/or the neural network of claim 9 and/or the database of claim 12.

15. A system comprising a processor coupled to a memory, the memory having recorded thereon the computer program of claim 13 and/or the neural network of claim 9 and/or the database of claim 12.

1000

1010

CPU

1070

RAM

1020

Mass storage
devices controler

Display

1080

1030

Hard
drive

Haptic
device

1090

Network Adapter

1050

Network

1060

B
U
S

Video
RAM

1100

GPU

1110

## FIG. 1

210

220

230

240

## FIG. 2

Stress, σ

Strain hardening

Necking

Ultimate strength

Fracture

Yield strength

Rise

Run

$$\text{Young's modulus} = \text{Slope} = \frac{\text{Rise}}{\text{Run}}$$

Strain, ε

## FIG. 3

FIG. 4

(a) vf = 0.2    (b) vf = 0.35 (test)    (c) vf = 0.5    (d) vf = 0.65 (test)    (e) vf = 0.8

FIG. 5

FIG. 6

FIG. 7

FIG. 8

## FIG. 9

## FIG. 10

## FIG. 11

A simple neural network

input layer    hidden layer    output layer

## FIG. 12

$p$ $(w, \theta)$

$\mathbb{C}_1$

$\mathbb{C}_2$

$\overline{\mathbb{C}}$

DMN

## FIG. 13

Select the parameters to be identified → Define the value ranges for each parameter → Train the parametric DMN model with a DOE → Save the trained DMN to the database → Perform inverse identification

FIG. 14

## FIG. 15

$$\overline{\mathbb{C}}' = \mathrm{Lam}(\mathbb{C}'_1, \mathbb{C}'_2; f', \boldsymbol{\theta}')$$

$$f' = \frac{w'_1}{w'_1 + w'_2}$$

## FIG. 16

## FIG. 17

## FIG. 18

33

## FIG. 19

FIG. 20

## FIG. 21

## FIG. 22

FIG. 23

(b)

FIG. 24

(c)

FIG. 25

(a)  vf = 0.459     (b)  vf = 0.537 (test)     (c)  vf = 0.608     (d)  vf = 0.729

## FIG. 26

FIG. 27

FIG. 28

FIG. 29

(a)

(b)

(c)

FIG. 30

(a)

(b)

## FIG. 31

## FIG. 32

Europäisches Patentamt
European Patent Office
Office européen des brevets

# EUROPEAN SEARCH REPORT

**Application Number**

EP 23 30 6465

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEBASTIAN GAJEK ET AL: "An FE-DMN method for the multiscale analysis of fiber reinforced plastic components", ARXIV.ORG, CORNELL UNIVERSITY LIBRARY, 201 OLIN LIBRARY CORNELL UNIVERSITY ITHACA, NY 14853, 15 March 2021 (2021-03-15), XP081969549, DOI: 10.1016/J.CMA.2021.113952 * the whole document * | 1-15 | INV. G16C60/00 |
| X | HUANG TIANYU ET AL: "Microstructure-guided deep material network for rapid nonlinear material modeling and uncertainty quantification", COMPUTER METHODS IN APPLIED MECHANICS AND ENGINEERING, NORTH-HOLLAND, AMSTERDAM, NL, vol. 398, 27 June 2022 (2022-06-27), XP087130835, ISSN: 0045-7825, DOI: 10.1016/J.CMA.2022.115197 [retrieved on 2022-06-27] * the whole document * | 1-15 | |
| X | LIU ZELIANG ET AL: "Transfer learning of deep material network for seamless structure-property predictions", COMPUTATIONAL MECHANICS, SPRINGER, BERLIN, DE, vol. 64, no. 2, 12 April 2019 (2019-04-12), pages 451-465, XP036826309, ISSN: 0178-7675, DOI: 10.1007/S00466-019-01704-4 [retrieved on 2019-04-12] * the whole document * | 1-15 | TECHNICAL FIELDS SEARCHED (IPC) G16C G06N |

-/--

The present search report has been drawn up for all claims

| Place of search | Date of completion of the search | Examiner |
|---|---|---|
| The Hague | 14 February 2024 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches Patentamt

European Patent Office

Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | LI TIANYI: "Micromechanics-informed parametric deep material network for physics behavior prediction of heterogeneous materials with a varying morphology", COMPUTER METHODS IN APPLIED MECHANICS AND ENGINEERING, vol. 419, 13 December 2023 (2023-12-13), page 116687, XP093130333, AMSTERDAM, NL ISSN: 0045-7825, DOI: 10.1016/j.cma.2023.116687 * the whole document * ----- | 1-15 | |

TECHNICAL FIELDS
SEARCHED     (IPC)

The present search report has been drawn up for all claims

| Place of search | Date of completion of the search | Examiner |
|---|---|---|
| The Hague | 14 February 2024 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

**REFERENCES CITED IN THE DESCRIPTION**

**Non-patent literature cited in the description**

- **LIU** ; **WU** ; **KOISHI**. A deep material network for multiscale topology learning and accelerated nonlinear modeling of heterogeneous materials. *Computer Methods in Applied Mechanics and Engineering*, 2019, vol. 345, 1138-1168

- **LIU et al.** A deep material network for multiscale topology learning and accelerated nonlinear modeling of heterogeneous materials. *Computer Methods in Applied Mechanics and Engineering*, 2019, vol. 345, 1138-1168

- **LIU et al.** A deep material network for multiscale topology learning and accelerated nonlinear modeling of heterogeneous materials. *Computer Methods in Applied Mechanics and Engineering*, 2019, vol. 345, 1138-116